(19) Europäisches Patentamt
European Patent Office
Office européen des brevets

(11) Veröffentlichungsnummer: **0 236 872 B1**

# (12) EUROPÄISCHE PATENTSCHRIFT

(45) Veröffentlichungstag der Patentschrift: **25.11.92**

(21) Anmeldenummer: **87102771.0**

(22) Anmeldetag: **26.02.87**

(51) Int. Cl.5: **C07K 5/06**, C07C 235/00, C07C 233/00, C07C 237/00, C07C 239/00, A61K 37/02, A61K 31/16

## (54) Hydroxylaminderivate, deren Herstellung und Verwendung für Heilmittel.

(30) Priorität: **11.03.86 GB 8605977**
**12.12.86 GB 8629712**

(43) Veröffentlichungstag der Anmeldung:
**16.09.87 Patentblatt 87/38**

(45) Bekanntmachung des Hinweises auf die Patenterteilung:
**25.11.92 Patentblatt 92/48**

(84) Benannte Vertragsstaaten:
**AT BE CH DE FR GB IT LI LU NL SE**

(56) Entgegenhaltungen:
**EP-A- 0 075 896**
**EP-A- 0 082 088**
**EP-A- 0 126 974**

**CHEMICAL ABSTRACTS, Band 88, Nr. 20, 15. Mai 1978, Seite 223, Nr. 148033a, Columbus, Ohio, US; N. NISHINO et al.: "Peptide hydroxamates as inhibitors of thermolysin and related metalloproteases", & PET., PROC. AM. PEPT. SYMP., 5TH 1977, 236-8**

(73) Patentinhaber: **F. HOFFMANN-LA ROCHE AG**
**Postfach 3255**
**CH-4002 Basel(CH)**

(72) Erfinder: **Handa, Balraj Krishnan**
**89 Lords Wood**
**Welwyn Garden City Herts.(GB)**
Erfinder: **Johnson, William Henry**
**87 West Hill**
**Hitchin, Herts.(GB)**
Erfinder: **Machin, Peter James**
**42 Parklands Road**
**London SW16 6TF(GB)**

(74) Vertreter: **Grossner, Lutz, Dr. et al**
**Grenzacher Strasse 124 Postfach 3255**
**CH-4002 Basel(CH)**

EP 0 236 872 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 106, Nr. 7, 16. Februar 1987, Seite 34, Nr. 43617f, Columbus, Ohio, US; G. DI PASOUALE et al.: "Proteoglycan- and collagen-degrading enzymes from human interleukin 1-stimulated chondrocytes from several species: proteoglycanase and collagenase inhibitors as potentially new disease-modifying antiarthritic agents", & PROC. SOC. EXP. BIOL. MED. 1986, 183(2) 262-7

## Beschreibung

Die vorliegende Erfindung betrifft Hydroxylaminderivate, ein Verfahren zu deren Herstellung und Heilmittel, die diese Derivate enthalten.

Die erfindungsgemässen Hydroxylaminderivate besitzen eine andere Struktur als die ebenfalls eine N-Hydroxyaminocarboxylgruppe oder eine N-Formyl-N-hydroxyaminogruppe enthaltenden, z.B. in den EP-A-75896, 82088 und 126974 und in CA 88:148033a beschriebenen Hydroxyaminderivaten. Erstere die allgemeinen Formel

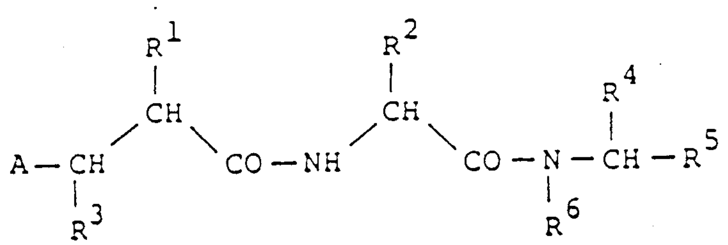

I

worin

| | |
|---|---|
| A | eine Gruppe der Formel HN(OH)-CO- oder HCO-N(OH)-; |
| $R^1$ | eine $C_2$-$C_5$-Alkylgruppe; |
| $R^2$ | die charakterisierende Gruppe einer natürlichen $\alpha$-Aminosäure ist, in der gegebenenfalls vorliegende funktionelle Gruppen geschützt sein können, Aminogruppen acyliert sein können und Carboxylgruppen amidiert sein können, mit der Bedingung, dass $R^2$ nicht Wasserstoff oder eine Methylgruppe darstellt; |
| $R^3$ | Wasserstoff, eine Amino-, Hydroxy-, Mercapto-, $C_1$-$C_6$-Alkyl-, $C_1$-$C_6$-Alkoxy-, $C_{1-6}$-Alkylamino-, $C_1$-$C_6$-Alkylthio- oder Aryl-($C_1$-$C_6$-alkyl)-Gruppe oder Amino-($C_1$-$C_6$-alkyl)-, Hydroxy-($C_1$-$C_6$-alkyl)-, Mercapto-($C_1$-$C_6$-alkyl)- oder Carboxy-($C_1$-$C_6$-alkyl)-Gruppe darstellt, worin die Amino-, Hydroxy-, Mercapto- oder Carboxylgruppe geschützt sein und die Aminogruppe acyliert oder die Carboxylgruppe amidiert sein kann; |
| $R^4$ | Wasserstoff oder eine Methylgruppe; |
| $R^5$ | Wasserstoff oder eine $C_1$-$C_6$-Alkyl, $C_1$-$C_6$-Alkoxy-$C_1$-$C_6$-alkyl, Di($C_1$-$C_6$-alkoxy)methylen, Carboxy, ($C_1$-$C_6$-Alkyl)carbonyl, ($C_1$-$C_6$-Alkoxy)carbonyl, Arylmethoxycarbonyl, ($C_1$-$C_6$-Alkyl)aminocarbonyl oder Arylaminocarbonylgruppe; und |
| $R^6$ | Wasserstoff oder eine Methylgruppe darstellt; oder |
| $R^2$ und $R^4$ | zusammen eine Gruppe $-(CH_2)_n-$ darstellen, worin n eine Zahl von 4-11 bedeutet; oder |
| $R^4$ und $R^5$ | zusammen eine Trimethylengruppe darstellen; |

und pharmazeutisch anwendbare Salze derjenigen Verbindungen, die sauer oder basisch sind.

Der hier allein oder in Kombination verwendete Ausdruck "Alkyl" bedeutet geradkettige oder verzweigtkettige gesättigte Kohlenwasserstoffgruppen, die die jeweils angegebene Anzahl von Kohlenstoffatomen enthalten. Beispiele von Alkylgruppen sind Methyl, Aethyl, n-Propyl, Isopropyl, n-Butyl, sek.Butyl, Isobutyl, tert.Butyl und n-Pentyl. Der Ausdruck "Alkoxy" bedeutet Alkyläthergruppen, worin der Ausdruck "Alkyl" die obige Bedeutung hat. Beispiele sind Methoxy, Aethoxy, n-Propoxy, Isopropoxy, n-Butoxy und tert.Butoxy. Der Ausdruck "Aryl" im Zusammenhang mit "Aryl-($C_1$-$C_6$-alkyl)", "Arylmethoxycarbonyl" und "Arylaminocarbonyl" bedeutet eine Phenylgruppe, die gewünschtenfalls mit einem oder mehreren Substituenten wie $C_1$-$C_6$-Alkyl, $C_1$-$C_6$-Alkoxy, Halogen, Trifluormethyl, etc. substituiert ist.

Der Ausdruck "charakterisierende Gruppe einer natürlichen $\alpha$-Aminosäure" bedeutet die Gruppe R in einer $\alpha$-Aminosäure der Formel $H_2N$-CH(R)-COOH, die natürlich vorkommt. So kann unter Berücksichtigung der oben in Bezug auf $R^2$ erwähnten Bedingung die charakterisierende Gruppe eine der folgenden sein, wobei die entsprechenden natürliche $\alpha$-Aminosäure in Klammern nachgestellt ist: Isopropyl (Valin), Isobutyl (Leucin), Benzyl (Phenylalanin), p-Hydroxybenzyl (Tyrosin), Hydroxymethyl (Serin), Mercaptomethyl (Cystein), 1-Hydroxyäthyl (Threonin), 2-Methylthioäthyl (Methionin), Carboxymethyl (Asparaginsäure), 2-Carboxyäthyl (Glutaminsäure), 3-Guanidinopropyl (Arginin), 4-Aminobutyl (Lysin) usw.

Eine in $R^2$ anwesende funktionelle Gruppe und eine Amino-, Hydroxy-, Mercapto- oder Carboxygruppe, die in $R^3$ vorliegt, kann in der Peptidchemie an sich bekannten Weise gescüßhützt sein. Beispielsweise kann eine Hydroxygruppe in Form eines leicht spaltbaren Aethers wie des tert.Butyl, Benzyl oder

Tetrahydropyranäthers oder in Form eines leicht spaltbaren Esters wie des Acetats vorliegen. Eine Mercaptogruppe kann beispielsweise durch eine tert.Butyl- oder Benzylgruppe geschützt sein. Eine Aminogruppe kann beispielsweise durch eine tert.Butoxycarbonyl-, Benzyloxycarbonyl-, Formyl-, Trityl-, Trifluoracetyl-, 2-(Biphenylyl)isopropoxycarbonyl- oder Isobornyloxycarbonylgruppe oder in Form einer Phthalimidogruppe geschützt sein. Eine Carboxygruppe kann beispielsweise in Form eines leicht spaltbaren Esters wie des Methyl-, Aethyl-, tert.Butyl- oder Benzylester geschützt sein.

Eine in $R^2$ und/oder $R^3$ anwesende Aminogruppe kann durch eine nicht-spaltbare Acylgruppe acyliert sein; beispielsweise durch eine Acylgruppe, die von einer aromatischen Dicarbonsäure oder einer Aminocarbonsäure abgeleitet ist. Beispiele solcher aromatischen Dicarbonsäuren sind Benzoldicarbonsäuren wie Phhthalsäure und Terephthalsäure. Beispiele solcher Aminocarbonsäuren sind $\alpha$-Aminosäuren wie natürliche Aminosäuren, z.B. Glycin und Alanin.

Eine in $R^2$ und/oder $R^3$ anwesende Carboxylgruppe kann in üblicher Weise amidiert sein. Beispiele von amidierten Carboxylgruppen sind Aminocarbonyl, ($C_1$-$C_6$-Alkyl)aminocarbonyl, Di($C_1$-$C_6$-Alkyl)-aminocarbonyl oder Arylaminocarbonylgruppen, sowie Carboxylgruppen, die mit einer Aminocarbonsäure wie einer natürlichen Aminocarbonsäure, z.B. Glycin oder Alanin, amidiert sind.

Diejenigen Verbindungen der Formel I die basisch sind, bilden pharmazeutisch anwendbare Salze mit anorganischen Säuren, z.B. Halogenwasserstoffsäuren wie Salzsäure und Bromwasserstoffsäure, Schwefelsäure, Salpetersäure, Phosphorsäure und mit organischen Säuren wie Essigsäure, Weinsäure, Bernsteinsäure, Fumarsäure, Maleinsäure, Aepfelsäure, Salicylsäure, Methansulfonsäure und p-Toluolsulfonsäure.

Diejenigen Verbindungen der Formel I die sauer sind, bilden pharmazeutisch anwendbare Salze mit Basen wie Alkalimetallhydroxyden, z.B. Natriumhydroxyd und Kaliumhydroxyd, Erdalkalimetallhydroxyden, z.B. Calciumhydroxyd und Magnesiumhydroxyd, oder Ammoniumhydroxyd.

Die Verbindungen der Formel I enthalten mindestens zwei asymmetrische Kohlenstoffatome und können infolgedessen als optisch aktive Enantiomere, als Diastereoisomere oder als Racemate vorliegen.

In der obigen Formel I bedeutet $R^1$ vorzugsweise eine $C_3$- oder $C_4$-Alkylgruppe, besonders eine n-Propyl-, Isobutyl- oder sek.Butylgruppe. $R^2$ stellt vorzugsweise eine Isopropyl- oder Isobutylgruppe, eine geschützte p-Hydroxybenzylgruppe, insbesondere eine p-Methoxybenzylgruppe oder eine geschützte oder acylierte 4-Aminobutylgruppe, besonders eine 4-tert.Butoxycarbonylaminobutyl-, 4-Glycylaminobutyl-oder 4-(4-Carboxybenzoylamino)butylgruppe dar. $R^3$ stellt vorzugsweise Wasserstoff, eine Methylgruppe oder eine geschützte 4-Aminobutylgruppe, besonders Wasserstoff, eine Methylgruppe oder eine 4-Phthaloylaminobutylgruppe dar. Vorzugsweise stellt $R^4$ Wasserstoff oder eine Methylgruppe oder $R^2$ und $R^4$ zusammen eine Gruppe der Formel $-(CH_2)_n-$ dar, worin n eine Zahl von 5-9 ist. Wenn $R^4$ Wasserstoff oder eine Methylgruppe darstellt, ist $R^5$ vorzugsweise Wasserstoff oder $C_1$-$C_6$-Alkyl, $C_1$-$C_6$-Alkoxy-$C_1$-$C_6$-alkyl, Di($C_1$-$C_6$-Alkoxy)methylen, Carboxyl oder ($C_1$-$C_6$-Alkoxy)carbonyl, insbesondere Carboxyl oder ($C_1$-$C_6$-Alkoxy)-carbonyl, wobei die bevorzugte ($C_1$-$C_6$-Alkoxy)carbonylgruppe eine Aethoxycarbonylgruppe ist. Wenn $R^2$ und $R^4$ zusammen eine Gruppe der Formel $-(CH_2)_n-$ darstellen, ist $R^5$ vorzugsweise Wasserstoff. $R^6$ ist vorzugsweise Wasserstoff.

Die besonders bevorzugten Verbindungen der vorliegenden Erfindung sind somit diejenigen, in denen $R^1$ n-Propyl, Isobutyl oder sek.Butyl, $R^2$ Isopropyl, Isobutyl, p-Methoxybenzyl, 4-tert.Butoxycarbonylamino-butyl, 4-Glycylamino-butyl oder 4-(4-Carboxybenzoylamino)butyl, $R^3$ Wasserstoff oder Methyl oder 4-Phthaloylamino-butyl, $R^4$ Wasserstoff oder Methyl, $R^5$ Carboxyl oder Aethoxycarbonyl und $R^6$ Wasserstoff oder $R^2$ und $R^4$ zusammen $-(CH_2)_n-$ mit n = 5-9, $R^5$ Wasserstoff und $R^6$ Wasserstoff bedeuten.

Das Kohlenstoffatom, an das $R^1$ gebunden ist, hat vorzugsweise (R)-Konfiguration. Wenn $R^2$ die charakterisierende Gruppe einer natürlichen $\alpha$-Aminosäure gemäss vorstehender Definition bedeutet, ist dies vorzugsweise eine L-Aminosäure. Das Kohlenstoffatom, an das $R^3$ gebunden ist, hat (R)- oder (S)-Konfiguration je nach der Art des Substituenten $R^3$. Die bevorzugte Stereochemie an diesem Kohlenstoffatom ist (S), wenn $R^3$ $C_1$-$C_6$-Alkyl ist. Das Kohlenstoffatom, an das $R^4$ gebunden ist, hat vorzugsweise (S)-Konfiguration, wenn $R^4$ Methyl und $R^5$ Carboxyl, ($C_1$-$C_6$-Alkoxy)carbonyl, Arylmethoxycarbonyl, ($C_1$-$C_6$-Alkyl)aminocarbonyl oder Arylcarbonylamino ist.

Besonders bevorzugte erfindungsgemässe Hydroxylaminderivate sind:

[4-(N-Hydroxyamino) 2(R)-isobutylsuccinyl]-L-leucylglycinäthylester;

[4-(N-Hydroxyamino) 2(R)-isobutylsuccinyl]-L-leucyl-L-alaninäthylester;

[N-Formyl-N-hydroxy 2(RS)-isobutyl-3-aminopropionyl]-L-leucylglycinäthylester;

$N^{\alpha}$-[4-(N-Hydroxyamino) 2(R)-isobutylsuccinyl]-$N^{\epsilon}$-glycyl-L-lysyl-L-alaninäthylester;

[4-(N-Hydroxyamino) 2(R)-isobutylsuccinyl]-3(RS)-aminolaurolactam und

[4-(N-Hydroxyamino) 3(S)-phthaloylaminobutyl-2(R)-isobutylsuccinyl]-L-leucylglycinäthylester.

Beispiele weiterer hervorzuhebender erfindungsgemässer Hydroxylaminderivate sind:

[4-(N-Hydroxyamino) 2(RS)-isobutylsuccinyl]-L-leucylglycinäthylester;

[4-(N-Hydroxyamino) 2(RS)-isobutylsuccinyl]-L-leucylglycinisopentylamid;
[4-(N-Hydroxyamino) 2(RS)-isobutylsuccinyl]-L-valylglycinäthylamid;
[4-(N-Hydroxyamino) 2(RS)-isobutylsuccinyl]-L-leucylglycinäthylamid;
$N^{\alpha}$-[4-(N-Hydroxyamino) 2(RS)-isobutylsuccinyl]-$N^{\varepsilon}$-tert.butoxycarbonyl-L-lysylglycinäthylamid;
[4-(N-Hydroxyamino) 2(RS)-isobutylsuccinyl]-O-methyl-L-tyrosinylglycinäthylester;
[4-(N-Hydroxyamino) 2(RS)-isobutylsuccinyl]-O-methyl-L-tyrosinylglycinäthylamid;
[4-(N-Hydroxyamino) 2(RS)-isobutylsuccinyl]-L-leucyl-L-alaninäthylester;
[4-(N-Hydroxyamino) 2(RS)-isobutylsuccinyl]-L-leucinpentylamid;
[4-(N-Hydroxyamino) 2(RS)-isobutylsuccinyl]-L-leucylglycinisopentylester;
[4-(N-Hydroxyamino) 2(RS)-isobutylsuccinyl]-L-leucinmethylamid;
[4-(N-Hydroxyamino) 2(R)-propylsuccinyl]-L-leucylglycinäthylester;
[4-(N-Hydroxyamino) 2(RS)-sec.butylsuccinyl]-L-leucylglycinäthylester;
[4-(N-Hydroxyamino) 2(R)-isobutylsuccinyl]-L-leucyl-L-alanin;
[4-(N-Hydroxyamino) 2(RS)-isobutylsuccinyl]-L-leucylglycinmethylester;
[4-(N-Hydroxyamino) 2(RS)-isobutylsuccinyl]-L-leucylsarcosinäthylester;
[4-(N-Hydroxyamino) 2(RS)-isobutylsuccinyl]-L-leucyl-L-prolinäthylester;
[4-(N-Hydroxyamino) 2(RS)-isobutylsuccinyl]-L-leucyl-L-alaninisopropylester;
[4-(N-Hydroxyamino) 2(RS)-isobutylsuccinyl]-L-leucinisopropylamid;
[4-(N-Hydroxyamino) 2(RS)-isobutylsuccinyl]-L-leucin-2-oxopropylamid;
[4-(N-Hydroxyamino) 2(RS)-isobutylsuccinyl]-L-leucin-2-methoxyäthylamid;
[4-(N-Hydroxyamino) 2(RS)-isobutylsuccinyl]-L-leucin-2,2-dimethoxyäthylamid;
$N^{\alpha}$[4-(N-Hydroxyamino) 2(R)-isobutylsuccinyl]-$N^{\varepsilon}$-glycyl-L-lysinmethylamid;
$N^{\alpha}$-[4-(N-Hydroxyamino) 2(R)-isobutylsuccinyl]-$N^{\varepsilon}$-(4-carboxybenzoyl)-L-lysyl-L-alaninäthylester;
$N^{\alpha}$-[4-(N-Hydroxyamino) 2(R)-isobutylsuccinyl]-$N^{\varepsilon}$-(4-carboxybenzoyl)-L-lysyl-L-alanin;
[4-(N-Hydroxyamino) 2(R)-isobutylsuccinyl]-3(RS)-amino-octahydro-2H-azonin-2-on;
[4-(N-Hydroxyamino) 3(S)-methyl-2(R)-isobutylsuccinyl]-L-leucylglycinäthylester;
[N-Formyl-N-hydroxy 2(RS)-isobutyl-3-aminopropionyl]-L-leucyl-L-alaninäthylester und
[N-Formyl-N-hydroxy 2(RS)-isobutyl-3-aminopropionyl]-L-leucinmethylamid.

Die Verbindungen der vorliegenden Erfindung und deren pharmazeutisch anwendbaren Salze von dejenigen Verbindungen, die sauer oder basisch sind, können erfindungsgemäss durch Hydrogenolyse oder Hydrolyse einer Verbindung der Formel

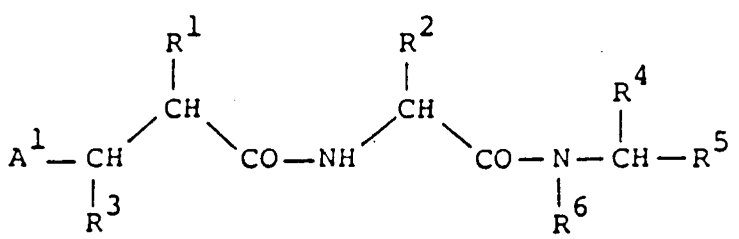

II

worin $A^1$ eine Gruppe der Formel $HN(R^7)$-CO- oder HCO-$N(R^7)$- darstellt, worin $R^7$ eine durch Hydrogenolyse oder Hydrolyse in eine Hydroxygruppe umwandelbare Gruppe ist und $R^1$, $R^2$, $R^3$, $R^4$, $R^5$ und $R^6$ die oben gegebene Bedeutung haben,
und, gewünschtenfalls, Umwandlung eines sauren oder basischen Produkts in ein pharmazeutisch anwendbares Salz hergestellt werden.

Der Rest $R^7$ in Verbindungen der Formel II kann jede Gruppe sein, die in eine Hydroxygruppe durch Hydrolyse oder Hydrogenolyse umgewandelt werden kann. Beispiele solcher Gruppen sind Benzyloxy, Tetrahydropyranyloxy, Benzhydryloxy, Trityloxy und tert.Butoxy.

Die Umwandlung einer durch $R^7$ dargestellten Gruppe in eine Hydroxygruppe durch Hydrogenolyse kann in an sich bekannter Weise unter Verwendung eines für solche Zwecke üblichen Hydrierkatalysators, z.B. eines Palladiumkatalysators vorgenommen werden. Die Hydrogenolyse wird unter allgemeinen bekannten Bedingungen durchgeführt, beispielsweise in einem inerten organischen Lösungsmittel wie einem Alkanol, z.B. Methanol oder Aethanol, oder in Dimethylformamid bei Raumtemperatur und unter Atmosphärendruck. Vorzugsweise ist $R^7$ eine Benzyloxygruppe.

Die Umwandlung einer durch $R^7$ dargestellten Gruppe in eine Hydroxygruppe durch Hydrolyse kann ebenfalls in an sich bekannter Weise durch Behandlung mit einer organischen oder anorganischen Säure

vorgenommen werden. Beispiele von solchen Säuren sind Alkancarbonsäuren wie Essigsäure, Trifluoressigsäure, aromatische Sulfonsäuren wie p-Toluolsulfonsäure und Mineralsäuren wie Schwefelsäure oder Halogenwasserstoffsäuren, z.B. Salzsäure oder Bromwasserstoffsäure. Die Hydrolyse wird in üblicher Weise durchgeführt, beispielsweise in wässrigem Medium oder in einem organischen Lösungsmittel bei Raumtemperatur und Atmosphärendruck. Wenn die Hydrolyse unter Verwendung einer organischen Säure ausgeführt wird, kann diese selbst als Lösungsmittel dienen. Vorzugsweise ist $R^7$ eine Tetrahydropyranylgruppe, wobei die Hydrolyse in wässrigem Medium durchgeführt wird.

Die Umwandlung einer resultierenden Verbindung der Formel I, die sauer oder basisch ist, in ein pharmazeutisch anwendbares Salz, kann in an sich bekannter Weise durch Behandlung mit der entsprechenden Base bzw. Säure bewerkstelligt werden.

Die Verbindungen der Formel II, die als Ausgangsmaterial im erfindungsgemässen Verfahren dienen, sind neue Verbindung und als solche ebenfalls Gegenstand der Erfindung.

Diejenigen Verbindungen der Formel II, in denen $A^1$ eine Gruppe der Formel $HN(R^7)$-CO- darstellt, können beispielsweise dadurch hergestellt werden, dass man eine Verbindung der allgemeinen Formel

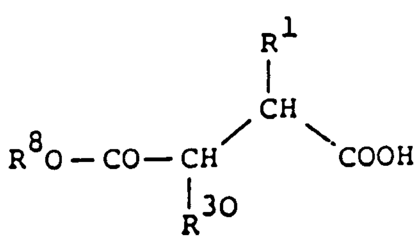

III

worin $R^1$ die obige Bedeutung hat, $R^8$ eine Schutzgruppe darstellt und $R^{30}$ dasselbe wie $R^3$ darstellt, wobei eine Amino, Hydroxy, Mercapto oder Carboxygruppe in geschützter Form vorliegt,
mit einer Verbindung der allgemeinen Formel

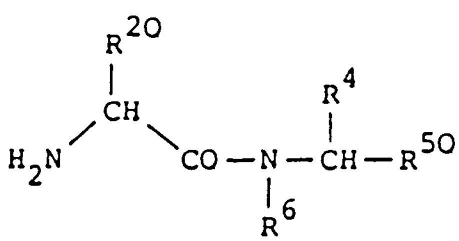

IV

worin $R^4$ und $R^6$ die obige Bedeutung haben, $R^{20}$ die gleiche Bedeutung wie $R^2$ hat, wobei eine anwesende funktionelle Gruppe geschützt ist und $R^{50}$ die gleiche Bedeutung wie $R^5$ hat, wobei eine Carboxylgruppe geschützt ist,
umsetzt, die Schutzgruppe $R^8$ aus der erhaltenen Verbindung der allgemeinen Formel

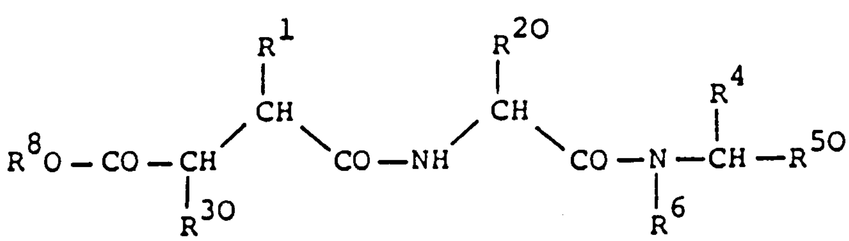

V

worin $R^1$, $R^4$, $R^6$, $R^8$, $R^{20}$, $R^{30}$ und $R^{50}$ die obige Bedeutung haben,
abspaltet, die erhaltene Verbindung der allgemeinen Formel

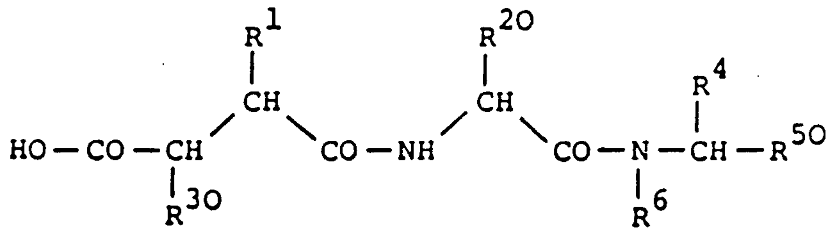

VI

worin $R^1$, $R^4$, $R^6$, $R^{20}$, $R^{30}$ und $^{50}$ die obige Bedeutung haben,
mit einer Verbindung der allgemeinen Formel

$R^7$-$NH_2$ VII

worin $R^7$ die obige Bedeutung hat,
umsetzt und gewünschtenfalls in beliebiger Reihenfolge eine in $R^{20}$ und/oder $R^{30}$ anwesende Schutzgruppe abspaltet und eine durch $R^{50}$ dargestellte geschützte Carboxylgruppe in eine Carboxylgruppe umwandelt.

Es versteht sich, dass in dem oben beschriebenen Verfahren zur Herstellung von Verbindung der Formel II, in denen $A^1$ eine Gruppe der Formel HN($R^7$)-CO- darstellt, eine in den Verbindungen der Formeln IV und III in den Substituenten $R^{20}$ und $R^{30}$ anwesende Schutzgruppe bzw. eine Schutzgruppe in $R^{50}$ so beschaffen sein muss, dass sie nicht unter den gleichen Bedingungen wie die durch $R^8$ bezeichnete Schutzgruppe abgespalten wird.

Der Substituent $R^8$ in der Formel III kann jede konventionelle Carboxylschutzgruppe darstellen, beispielsweise eine Methyl-, Aethyl-, tert.Butyl- oder Benzylgruppe.

Die Kondensation einer Verbindung der Formel III mit einer Verbindung der Formel IV kann auf in der Peptidchemie an sich bekannte Weise durchgeführt werden. So kann beispielsweise die Kondensation nach der Säurehalogenid-, Säureazid- oder Säureanhydrid-Methode oder nach der Methode der aktivierten Amide, der gemischten Carbonsäureamide oder aktivierten Ester hergestellt werden.

Die Abspaltung der Schutzgruppe $R^8$ aus einer Verbindung der Formel V kann in an sich bekannter Weise, z.B. durch Hydrolyse, wie früher beschrieben, bewerkstelligt werden.

Die Kondensation einer Verbindung der Formel VI mit einer Verbindung der Formel VII kann in an sich bekannter Weise durchgeführt werden, beispielsweise wie früher im Zusammenhang mit der Kondensation einer Verbindung der Formel III mit einer Verbindung der Formel IV beschrieben.

Schutzgruppen, die in $R^{20}$ und/oder $R^{30}$ im Kondensationsprodukt vorliegen, können, falls erwünscht, mittels in der Peptidchemie an sich bekannter Methoden abgespalten werden. Weiterhin kann, wenn $R^{50}$ eine geschüzte Carboxylgruppe darstellt, diese gewünschtenfalls in an sich bekannter Weise in eine Carboxylgruppe übergeführt werden, beispielsweise durch Verseifung, Behandlung mit wasserfreier Trifluoressigsäure, oder Hydrogenolyse, je nach Art der Schutzgruppe.

Ein weiteres Verfahren zur Herstellung von Verbindungen der Formel II, in denen $A^1$ eine Gruppe der Formel HN($R^7$)-CO- und $R^2$ die wie früher definierte charakterisierende Gruppe einer natürlichen $\alpha$-Aminosäure darstellt, besteht darin, dass man eine Verbindung der Formel III mit einer Verbindung der allgemeinen Formel

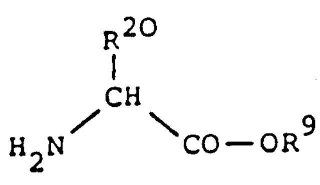

VIII

worin $R^{20}$ dieselbe Bedeutung wie $R^2$ hat, wobei eine anwesende funktionelle Gruppe geschützt ist und $R^9$ eine Carboxylschutzgruppe darstellt,
kondensiert, die durch $R^8$ dargestellte Schutzgruppe aus der erhaltenen Verbindung der Formel

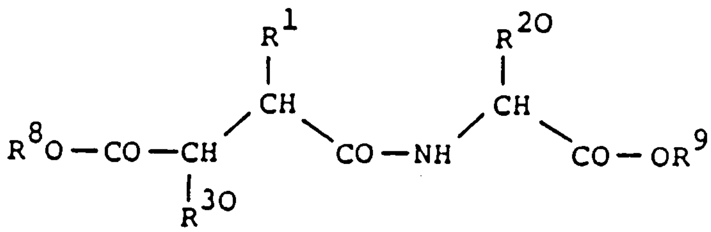

IX

worin $R^1$, $R^8$, $R^9$, $R^{20}$ und $R^3$ die obige Bedeutung haben,
abspaltet, die so erhaltene Verbindung der allgemeinen Formel

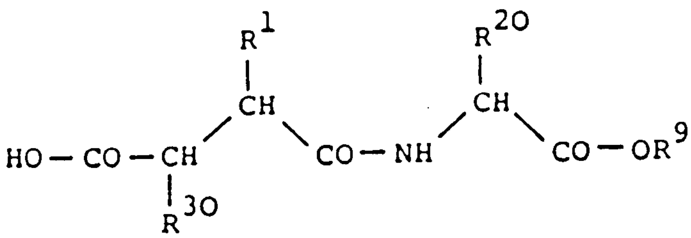

X

worin $R^1$, $R^9$, $R^{20}$ und $R^{30}$ die obige Bedeutung haben,
mit einer Verbindung der Formel VII kondensiert, die durch $R^9$ dargestellte Schutzgruppe aus der erhaltenen Verbindung der Formel

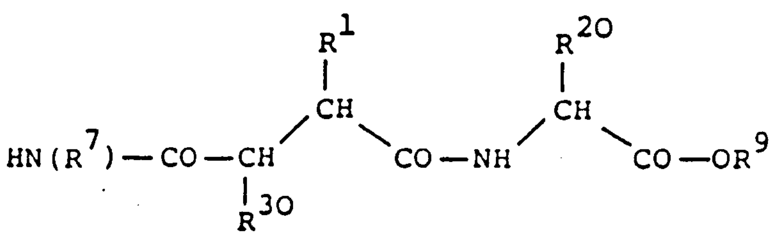

XI

worin $R^1$, $R^7$, $R^9$, $R^{20}$ und $R^{30}$ die obige Bedeutung haben,
abspaltet, die so erhaltene Verbindung der Formel

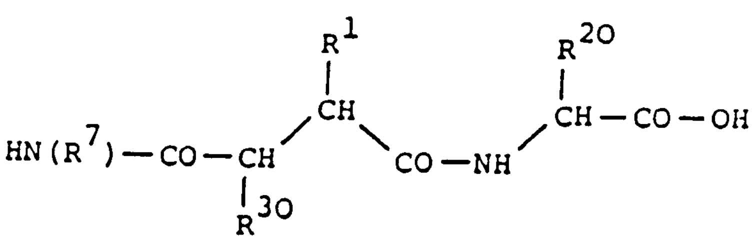

XII

worin $R^1$, $R^7$, $R^{20}$ und $R^{30}$ die obige Bedeutung haben,
mit einer Verbindung der allgemeinen Formel

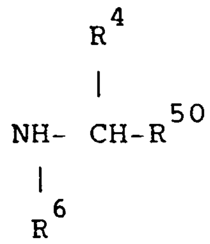

XIII

worin $R^4$, $R^6$ und $R^{50}$ die obige Bedeutung haben,
kondensiert und, gewünschtenfalls, in beliebiger Reihenfolge eine in $R^{20}$ und/oder $R^{30}$ anwesende Schutzgruppe abspaltet und eine durch $R^{50}$ bezeichnete geschützte Carboxylgruppe in eine Carboxylgruppe überführt.

In dem oben beschriebenen Verfahren versteht es sich wiederum, dass eine in $R^{20}$ und $R^{30}$ in den Verbindungen der Formeln VIII und III anwesende Schutzgruppe bzw. eine durch $R^9$ bezeichnete Schutzgruppe so beschaffen sein muss, dass sie nicht unter den gleichen Bedingungen wie die durch $R^8$ bezeichnete Gruppe abgespalten wird.

Die Kondensation einer Verbindung der Formel III mit einer Verbindung der Formel VIII kann in der gleichen Weise durchgeführt werden, wie früher im Zusammenhang mit der Kondensation einer Verbindung der Formel III mit einer Verbindung der Formel IV beschrieben.

Die Abspaltung der durch $R^8$ bezeichneten Gruppe aus einer Verbindung der Formel IX kann in gleicher Weise durchgeführt werden wie früher im Zusammenhang mit der Anspaltung der Gruppe $R^8$ aus einer Verbindung der Formel V beschrieben.

Die Kondensation einer Verbindung der Formel X mit einer Verbindung der Formel VII kann in der gleichen Weise ausgeführt werden, wie früher im Zusammenhang mit der Kondensation einer Verbindung der Formel III mit einer Verbindung der Formel IV beschrieben.

Die Abspaltung der durch $R^9$ bezeichneten Schutzgruppe aus einer Verbindung der Formel XI kann in an sich bekannter Weise ausgeführt werden, beispielsweise durch Verseifung, Behandlung mit wasserfreier Trifluoressigsäure, Hydrogenolyse, etc. je nach Art der Schutzgruppe.

Die Kondensation einer Verbindung der Formel XII mit einer Verbindung der Formel XIII kann in der gleichen Weise ausgeführt werden wie früher im Zusammenhang mit der Kondensation einer Verbindung der Formel III mit einer Verbindung der Formel IV beschrieben.

Die anschliessende fakultative Abspaltung von Schutzgruppen, die in $R^{20}$ und/oder $R^{30}$ anwesend sind, und die Umwandlung einer durch $R^5$ dargestellten geschützten Carboxylgruppe kann wie früher beschrieben bewerkstelligt werden.

Die Verbindungen der Formel II, in der $A^1$ eine Gruppe der Formel HCO-N($R^7$)- darstellt, können beispielsweise dadurch hergestellt werden, dass man eine Verbindung der Formel

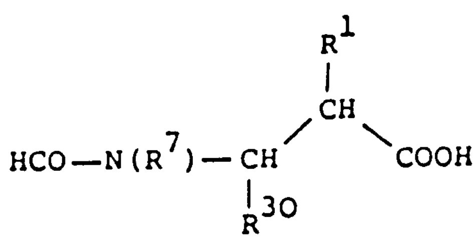

XIV

worin $R^1$, $R^7$ und $R^{30}$ die obige Bedeutung haben,
mit einer Verbindung der Formel IV kondensiert und, gewünschtenfalls, in beliebiger Reihenfolge eine in $R^{20}$ und/oder $R^{30}$ anwesende Schutzgruppe abspaltet und eine durch $R^{50}$ dargestellte geschützte Carboxylgruppe in eine Carboxylgruppe überführt.

Die Kondensation einer Verbindung der Formel XIV mit einer Verbindung der Formel IV kann in der gleichen Weise wie früher im Zusammenhang mit der Kondensation einer Verbindung der Formel III mit einer Verbindung der Formel IV beschreiben ausgeführt werden.

Die anschliessende fakultative Abspaltung von Schutzgruppen, die in $R^{20}$ und/oder $R^{30}$ vorliegen, und die Umwandlung einer durch $R^{50}$ dargestellten geschützten Carboxylgruppe in eine Carboxylgruppe können wie früher beschrieben durchgeführt werden.

Die Verbindungen der Formeln III, IV, VII, VIII, XIII und XIV können, soweit sie nicht bekannt oder Analoge bekannter Verbindungen sind, wie in den folgenden Beispielen beschrieben oder in Analogie dazu hergestellt werden.

Die erfindungsgemässen Hydroxylaminderivate besitzen Kollagenase-hemmende Aktivität und können in der Behandlung degenerativer Gelenkerkrankungen wie rheumatoider Arthritis oder Osteoarthritis verwendet werden.

In vitro kann die Hemmwirkung der vorliegenden Hydroxylaminderivate gegen Kollagenase, die aus Kulturen menschlicher Synovialfibroblasten erhalten wurde, nach der Methode von Dayer J-M et al., Proc. Natl. Acad. Sci. USA (1976), 73, 945 anhand des folgenden Testverfahrens gezeigt werden:

Prokollagenase aus Zellkulturmedium wurde partiell durch negative Adsorption an DEAE Sephadex A-50 bei pH 8,1 gereinigt und durch Behandlung mit Trypsin aktiviert. Die Hemmwirkung der vorliegenden Hydroxylaminderivate wurde unter Verwendung eines synthetischen Substrats, N-Acetyl-L-prolyl-L-leucyl glycyl-L-leucyl -L-leucylglycyläthylester für diese Kollagenase bestimmt. Das Enzym wurde einer Lösung von 0,5 mMol Substrat in 50 mMol Boratpuffer (pH 7,5), die 1 mMol Calciumchlorid und eine bekannte Konzentration des Peptidderivates enthielt, zugesetzt. Die erhaltene Lösung wurde 1 Stunde bei 37° C inkubiert. Die enzymatische Verdauung des Substrats wurde durch Zusatz eines gleichen Volumens von 2%iger (Gewicht/Volumen) Natriumhydrogencarbonatlösung in 50% wässrigem Methanol, das 0,1% (Gewicht/Volumen) Picrylsulfonsäure enthielt, beendet. Die erhaltene Lösung wurde eine weitere Stunde bei 37° C inkubiert, um eine Reaktion des durch Spaltung des Substrats erzeugten L-Leucyl-L-leucylglycyläthylesters und der Picrylsulfonsäure zu Trinitrophenyl-L-leucyl-L-leucylglycyläthylester zu ermöglichen. Die Reaktion wurde beendet und stabilisiert durch Ansäuern mit 1N Salzsäure. Die Konzentration an Trinitrophenyl-L-leucyl-L-leucylglycyläthylester wurde spektrophotometrisch bei 335 nm bestimmt. Die $IC_{50}$ ist diejenige Konzentration des Hydroxylaminderivats, die die Substratspaltung auf 50% des durch das Enzym allein erreichten Wertes reduziert.

Die in dem vorstehend beschriebenen Test mit repräsentativen erfindungsgemässen Hydroxylaminderivaten erhaltenen Resultate sind in der nachfolgenden Tabelle zusamengestellt:

Tabelle

| Hydroxylamin-derivat | Hemmwirkung gegen menschliche Synovialkollagenase $IC_{50}$ (Mol) |
|---|---|
| A | $2,5 \times 10^{-8}$ |
| B | $8,5 \times 10^{-9}$ |
| C | $3,0 \times 10^{-7}$ |
| D | $3,0 \times 10^{-8}$ |
| E | $3,8 \times 10^{-8}$ |
| F | $1,2 \times 10^{-8}$ |

A: [4-(N-Hydroxyamino) 2(R)-isobutylsuccinyl]-L-leucyl-glycinäthylester;

B: [4-(N-Hydroxyamino) 2(R)-isobutylsuccinyl]-L-leucyl-L--alaninäthylester;

C: [N-Formyl-N-hydroxy 2(RS)-isobutyl-3-aminopropionyl]--L-leucylglycinäthylester;

D: $N^{\alpha}$-[4-(N-Hydroxyamino) 2(R)-isobutylsuccinyl]-$-N^{\varepsilon}$-glycyl-L-lysyl-L-alaninäthylester;

E: [4-(N-Hydroxyamino) 2(R)-isobutylsuccinyl]-3(RS)--aminolaurolactam;

F: [4-(N-Hydroxyamino) 3(S)-phthaloylaminobutyl-2(R)-iso-butylsuccinyl]-L-leucylglycinäthylester.

Die erfindungsgemässen Hydroxylaminderivate können als Heilmittel in Form pharmazeutischer Präparate verwendet werden, die diese zusammen mit einem verträglichen pharmazeutischen Trägermaterial enthalten. Dieses Trägermaterial kann ein organisches oder anorganisches Trägermaterial sein, das für enterale oder parenterale Verabreichung geeignet ist, wie Wasser, Lactose, Stärke, Magnesiumstearat, Talkum, Gummi arabicum, Gelatine, Polyalkylenglykole und Vaseline. Die pharmazeutischen Präparate können in fester Form, z.B. als Tabletten, Pulver, Dragées, Suppositorien oder Kapseln oder in flüssiger Form, z.B. als Lösungen, Emulsionen oder Suspensionen vorliegen.

Falls nötig, können die pharmazeutischen Präparate üblichen pharmazeutischen Operationen wie Sterilisierung und ähnlichem unterworfen werden und können auch übliche pharmazeutische Hilfsstoffe wie Konservierungsmittel, Stabilisierungsmittel, Feuchthaltemittel, Salze zur Veränderung des osmotischen Druckes usw. enthalten. Die pharmazeutischen Präparate können auch andere therapeutisch wertvolle Substanzen enthalten.

Die pharmazeutischen Präparate können durch Vermischen eines erfindungsgemässen Hydroxylaminderivates mit einem verträgliche pharmazeutischen Trägermaterial und Verarbeitung in die gewünschte pharmazeutische Dosierungsform hergestellt werden.

Die erfindungsgemässen Hydroxylaminderivate können an Erwachsene in einem Dosierungsbereich von etwa 5 mg bis 30 mg, vorzugsweise etwa 10 mg bis 15 mg pro Tag verabreicht werden. Es versteht sich

jedoch, dass dieser Dosierungsbereich nur beispielhaft ist und nach oben und unten je nach der Potenz der verabreichten Verbindung, der zu behandelnden Erkrankung und den Bedürfnissen des Patienten variiert werden können.

Die folgenden Beispiele erläutern die Erfindung weiter. Die Struktur der erhaltenen Verbindung wurde durch NMR-Spektroskopie und Massenspektroskopie bestätigt. Die Zusammensetzung der Lösungsmittelsystem für die Dünnschichtchromatographie war wie folgt:

System A: Chloroform:Methanol:Essigsäure:Wasser 120:15:3:2.
System B: Chloroform:Methanol:Essigsäure:Wasser 60:18:2:3.
System C: Chloroform:Methanol:Essigsäure:Wasser 240:24:3:2.

Beispiel 1

0,5 g [4-(N-Benzyloxyamino 2(RS)-isobutylsuccinyl]-L-leucylglycinäthylester in 10 ml Aethanol wurden in Gegenwart von 0,1 g 5% Palladium/Kohle 1 Stunde hydriert. Das Gemisch wurde filtriert, das Filtrat eingedampft und der Rückstand unter vermindertem Druck getrocknet, wobei 0,38 g [4-(N-Hydroxyamino) 2(RS)-isobutylsuccinyl]-L-leucylglycinäthylester als Schaum erhalten wurden.

Der als Ausgangsmaterial verwendete [4-(N-Benzyloxyamino) 2(RS)-isobutylsuccinyl]-L-leucylglycinäthylester wurde wie folgt hergestellt:

(A) 49.4 g Kalium-tert.butoxyd in 350 ml tert.Butanol wurden unter Stickstoff und Rühren zum Rückfluss erhitzt. Im Verlauf von 0,25 Stunden wurden eine Gemisch von 36.4 ml Isobutyraldehyd und 83,1 ml Diäthylsuccinat zugesetzt und das Reaktionsgemisch 1,5 Stunden zum Rückfluss erhitzt. Die Lösung wurde auf ein kleines Volumen eingedampft, mit 2N Salzsäure angesäuert und weiter eingedampft. Der Rückstand wurde nach Verdünnen mit Wasser dreimal mit je 300 ml Diäthyläther extrahiert, die ätherischen Lösungen wurden vereinigt, mit Wasser gewaschen und dreimal mit je 200 ml 10% Kaliumcarbonatlösung extrahiert. Die basische Lösung wurde mit konzentrierter Salzsäure angesäuert, das Produkt mit Diäthyläther extrahiert, mit Wasser gewaschen, über Magnesiumsulfat getrocknet und zu einem Oel eingedampft. Das Oel wurde in 400 ml Aethanol gelöst und die Lösung 2 Stunden über 5 g 5% Palladium/Kohle hydriert. Nach Filtrieren und Eindampfen des Filtrates wurden 69,8 g rohes 1-Aethyl 2(RS)-isobutylhydrogensuccinat erhalten.

(B) Der vorstehende Aethylester wurde in 800 ml Dichlormethan, 800 ml Isobuten und 8 ml Schwefelsäure 5 Tage in einem verschlossenen Gefäss gerührt. Danach wurden 350 ml 5% Natriumhydrogencarbonatlösung zugesetzt und die Lösung eingedampft. Das Produkt wurde aus dem wässrigen Rückstand mit zwei 300 ml Portionen Diäthyläther extrahiert. Die ätherischen Extrakte wurden mit Wasser und gesättigter Natriumchloridlösung gewaschen, über Magnesiumsulfat getrocknet und eingedampft und lieferten 87,3 g 4-tert.Butyl 1-äthyl 2(RS)-isobutylsuccinat.

(C) Der im Abschnitt (B) hergestellte Diester wurde in 500 ml Aethanol, 380 ml Wasser und 120 ml 4N Natriumhydroxydlösung 16 Stunden bei Raumtemperatur gerührt. Das Aethanol wurde abgedampft, die Lösung mit 300 ml Wasser verdünnt, zweimal mit je 400 ml Diäthyläther extrahiert und mit konzentrierter Salzsäure angesäuert. Das Produkt wurde in Diäthyläther aufgenommen, mit Wasser und gesättigter Natriumchloridlösung gewaschen, über Magnesiumsulfat getrocknet und zu einem Oel eingedampft. Chromatographie an Silicagel mit Toluol/Essigsäure (19:1) als Elutionsmittel und anschliessendes Eindampfen und Entfernung von Spuren von Essigsäure durch Abdampfen mit Toluol lieferten 45,5 g 4-tert.Butyl 2(RS)-isobutylhydrogensuccinat als Oel, Rf (Toluol/Essigsäure 19:1) 0,32.

(D) 3,85 g N-Benzyloxycarbonyl-L-leucyl-glycinäthylester wurden 1 Stunde mit 10 ml 4N Bromwasserstoff in Essigsäure behandelt. Danach wurden 200 ml Diäthyläther zugesetzt und der Niederschlag durch zweifaches Dekantieren mit je 100 ml Diäthyläther gewaschen, unter vermindertem Druck getrocknet und in 50 ml Tetrahydrofuran gelöst. Die Lösung wurde auf 0°C gekühlt und mit 3,0 ml N-Aethylmorpholin neutralisiert. Danach wurden 2,3 g 4-tert.Butyl 2(RS)-isobutylhydrogensuccinat, 1,49 g Hydroxybenzotriazol und 2,26 g N,N′-Dicyclohexylcarbodiimid zugesetzt und das Gemisch 1 Stunde bei 0°C gerührt und dann über Nacht bei 0°C stehengelassen. Die Lösung wurde filtriert und das Filtrat eingedampft. Der Rückstand wurde in Aethylacetat aufgenommen und nacheinander mit Wasser, 5% Zitronensäurelösung, Wasser, 5% Natriumhydrogencarbonatlösung und gesättigter Kochsalzlösung gewaschen, über Magnesiumsulfat getrocknet und zu einem Oel eingedampft. Chromatographie an Silicagel mit Aethylacetat/Hexan (Gradient von 1:9 bis 1:1) lieferte 2,54 g [4-tert.Butyl 2(RS)-isobutylsuccinyl]-L-leucylglycinäthylester als Oel, Rf (Aethylacetat/Hexan 1:1) 0,73, 0.79.

(E) 2,54 g [4-tert.Butyl 2(RS)-isobutylsuccinyl]-L-leucylglycinäthylester wurden mit 5 ml 4N Bromwasserstoff in Essigsäure 0,25 Stunden behandelt. Die Lösung wurde eingedampft und das erhaltene Oel in Aethylacetat gelöst, mit Wasser und gesättigter Kochsalzlösung gewaschen, über Magnesiumsulfat

getrocknet und zu 2,1 g Oel eingedampft. Das Oel wurde in 30 ml Tetrahydrofuran aufgenommen, die Lösung auf -12° C gekühlt, mit 0,72 ml N-Aethylmorpholin und 0,74 ml Isobutylchloroformiat versetzt und 4 Minuten bei -12° C gerührt. Dann wurde eine Lösung von O-Benzylhydroxylamin (hergestellt aus 0,9 g O-Benzylhydroxylamin-hydrochlorid un 0,72 ml N-Aethylmorpholin in 5 ml Dimethylformamid bei 0° C) zugesetzt, das Gemisch 1 Stunde bei 0° C gerührt und dann über Nacht bei Raumtemperatur stehengelassen. Das Lösungsmittel wurde abgedampft und der Rückstand in Aethylacetat gelöst. Die Lösung wurde nacheinander mit Wasser, 5% Zitronensäurelösung, Wasser, 5% Natriumhydrogencarbonatlösung in gesättigter Kochsalzlösung gewaschen, über Magnesiumsulfat getrocknet und zu einem Oel eingedampft. Chromatographie an 75 g Silicagel mit Chloroform lieferte 1,73 g [4-(N-Benzyloxyamino) 2(RS)-isobutylsuccinyl]-L-leucylglycinäthylester als Oel.

Beispiel 2

0,28 g [4-(N-Benzyloxyamino) 2(RS)-isobutylsuccinyl-L-leucylglycinäthylester wurden durch HPLC auf einer Partesil (10 mikron) ODS-3-Säule mit Acetonitril/Wasser (1:1) mit einer Fliessgeschwindigkeit von 1,4 ml/Minute in die beiden Isomeren getrennt. Das erste Isomer ($R_t$ = 2,5 Minuten) (0,13 g) wurde 1 Stunde in 5 ml Aethanol über 20 mg 5% Palladium/Kohle hydriert. Das Gemisch wurde filtriert, das Filtrat eingedampft und der Rückstand unter vermindertem Druck getrocknet. Man erhielt 0,087 g [4-(N-Hydroxyamino) 2(R)-isobutylsuccinyl-L-leucylglycinäthylester als Schaum.

Beispiel 3

0,2 g [4-(N-Benzyloxyamino) 2(RS)-isobutylsuccinyl]-L-leucylglycin-isopentylamid wurden in 5 ml Aethanol in Gegenwart von 50 mg 5% Palladium/Kohle 1 Stunde hydriert. Das Gemisch wurde filtriert, das Filtrat eingedampft und der Rückstand unter vermindertem Druck getrocknet. Man erhielt 0,102 g [4-(N-Hydroxyamino) 2(RS)-isobutylsuccinyl]-L-leucylglycin-isopentylamid.

Da als Ausgangsmaterial verwendete [4-(N-Benzyloxyamino) 2(RS)-isobutylsuccinyl]-L-leucylglycin-isopentylamid wurde wie folgt hergestellt:

(A) 1,79 g N-Benzyloxycarbonyl-L-leucylglycin-isopentylamid wurden 1 Stunde mit 5 ml 4N Bromwasserstoff in Essigsäure behandelt. Danach wurden 150 ml Diäthyläther zugesetzt und das erhaltene L-Leucylglycin-isopentylamid-hydrobromid abfiltriert und unter vermindertem Druck getrocknet.

1,05 g 4-tert.Butyl 2(RS)-isobutylhydrogensuccinat in 10 ml Tetrahydrofuran wurden bei -12° C mit 0,58 ml N-Aethylmorpholin und 0,60 ml Isobutylchloroformiat versetzt und 4 Minuten bei -12° C gerührt. Eine Lösung des oben erhaltenen Hydrobromids in 5 ml Diemethylformamid wurde auf 0° C gekühlt und mit 0,9 ml N-Aethylmorpholin neutralisiert. Die beiden Lösungen wurden gemischt und das Gemisch 4 Stunden bei 0° C gerührt. Das Lösungsmittel wurde dann abgedampft und der Rückstand in Aethylacetat gelöst. Die Lösung wurde nacheinander mit 5% Zitronensäurelösung, Wasser, 5% Natriumhydrogencarbonatlösung und gesättigter Kochsalzlösung gewaschen, über Magnesiumsulfat getrocknet und eingedampft, wobei 2,04 g [4-tert.Butyl 2(RS)-isobutylsuccinyl]-L-leucylglycin-isopentylamid als Schaum erhalten wurden. Rf (Methanol/Chloroform 1:19) 0,59.

(B) 2,0 g [4-tert.Butyl 2(RS)-isobutylsuccinyl]-L-leucylglycin-isopentylamid wurden in 2 ml Aethylacetat aufgenommen und 2 Stunden mit 5 ml 4N Bromwasserstoff in Essigsäure behandelt. Das Gemisch wurde zur Trockene eingedampft und der Rückstand zur Entfernung von Spuren von Essigsäure mit Toluol abgedampft. Der Rückstand wurde in Dichlormethan aufgenommen und dreimal mit 5% Natriumhydrogencarbonatlösung extrahiert. Die wässrigen Lösungen wurden vereinigt und mit 2N Salzsäure angesäuert. Extraktion mit Dichlormethan, Waschen mit gesättigter Natriumchloridlösung und Eindampfen lieferte 1,2 g 2(RS)-Isobutylsuccinyl-L-leucylglycin-isopentylamid als Schaum. Die Säure wurde in 10 ml Tetrahydrofuran aufgenommen, die Lösung auf -12° C gekühlt, mit 0,37 ml N-Aethylmorpholin und 0,38 ml Isobutylchloroformiat versetzt und 4 Minuten bei -12° C gerührt. Danach wurden 0,36 g O-Benzylhydroxylamin in 2 ml Tetrahydrofuran zugesetzt und das Gemisch 2 Stunden bei 0° C gerührt. Das Lösungsmittel wurde abgedampft und der Rückstand in Aethylacetat gelöst. Die Lösung wurde nacheinander mit 5% Zitronensäurelösung, Wasser, 5% Natriumhydrogencarbonatlösung und gesättigter Kochsalzlösung gewaschen, über Magnesiumsulfat getrocknet und zu einem Oel eingedampft. Chromatographie an 50 g Silicagel mit 3% Methanol in Chloroform als Elutionsmittel lieferte 0,92 g [4-(N-Benzyloxyamino) 2(RS)-isobutylsuccinyl]-L-leucylglycin-isopentylamid als Schaum.

Beispiel 4

In analoger Weise wie im ersten Abschnitt von Beispiel 3 beschrieben wurde aus [4-(N-Benzyloxyamino) 2(RS)-isobutylsuccinyl]-L-valyglycinäthylamid das [4-(N-Hydroxyamino) 2(RS)-isobutylsuccinyl]-L-valylglycinäthylamid in einer Ausbeute von 83% erhalten.

Das als Ausgangsmaterial verwendete [4-(N-Benzyloxyamino) 2(RS)-isobutylsuccinyl]-L-valylglycinäthylamid wurde wie folgt hergestellt:

(A) In analoger Weise wie im Beispiel 3(A) beschrieben wurde aus N-Benzyloxycarbonyl-L-valylglycinäthylamid das [4-tert.Butyl 2(RS)-isobutylsuccinyl]-L-valylglycinäthylamid in einer Ausbeute von 92% erhalten. Rf (Methanol/Chloroform 1:9) 0,62.

In analoger Weise wie im Beispiel 3(B) beschrieben wurde aus [4-tert.Butyl 2(RS)-isobutylsuccinyl]-L-valylglycinäthylamid das [4-(N-Benzyloxyamino) 2(RS)-isobutylsuccinyl]-L-valylglycinäthylamidin einer Ausbeute von 42% erhalten.

Beispiel 5

0,2 g [4-(N-Benzyloxyamino) 2(RS)-isobutylsuccinyl]-L-leucylglycinäthylamid wurden in 5 ml Aethanol 1 Stunde in Gegenwart von 50 mg 5% Palladium/Kohle hydriert. Das Gemisch wurde filtriert, das Filtrat eingedampft und der Rückstand unter vermindertem Druck getrocknet. Man erhielt 0,16 g [4-(N-Hydroxyamino) 2(RS)-isobutylsuccinyl]-L-leucylglycinäthylamid als Schaum.

Das als Ausgangsmaterial verwendete [4-(N-Benzyloxyamino) 2(RS)-isobutylsuccinlyl]-L-leucylglycinäthylamid wurde in analoger Weise wie in den Beispielen 3(A) und 3(3B) beschrieben hergestellt.

Beispiel 6

2,56 g $N^{\alpha}$-[4-tert.Butyl 2(RS)-isobutylsuccinyl]-$N^{\epsilon}$-tert.butyloxycarbonyl -L-lysylglycinäthylamid wurden 3 Stunden mit 4N Chlorwasserstoff in Aethylacetat behandelt. Das Lösungsmittel wurde abgedampft, Toluol zugesetzt und das Gemisch zu einem Schaum eingedampft. Der Schaum wurde in 20 ml Dioxan und 20 ml 2N Natriumhydroxydlösung aufgenommen und das Gemisch bei 0°C 16 Stunden mit 4,3 g Di-tert.Butyldicarbonat behandelt. Das Dioxan wurde abgedampft, die Lösung mit 20 ml Wasser verdünnt, mit Aethylacetat extrahiert und mit 2N Salzsäure auf pH 3 angesäuert. Das Produkt wurde in Aethylacetat aufgenommen, mit Wasser und gesättigter Natriumchloridlösung gewaschen, über Magnesiumsulfat getrocknet und eingedampft. Man erhielt $N^{\alpha}$-[2-(RS)-Isobutylsuccinyl] -$N^{\epsilon}$-tert.butyloxycarbonyl-L-lysylglycinäthylamid in Form eines Oels. Die Säure wurde an O-Benzylhydroxylamin nach dem Verfahren von Beispiel 3(B) gekuppelt und lieferte 1,04 g $N^{\alpha}$-[4-(N-Benzyloxyamino) 2(RS)-isobutylsuccinyl]-$N^{\epsilon}$-tert.butyloxycarbonyl -L-lysylglycinäthylamid; Rf (Chloroform/Methanol 9:1) 0,46. Die Benzylschutzgruppe wurde wie im ersten Absatz von Beispiel 3 beschrieben entfernt, wobei $N^{\alpha}$-[4-(N-Hydroxyamino) 2(RS)-isobutylsuccinyl]-$N^{\epsilon}$-tert.butoxycarbonyl -L-lysylglycinäthylamid in 86% Ausbeute als Schaum erhalten wurde.

Beispiel 7

In analoger Weise wie in Beispiel 3 beschrieben wurde [4-(N-Hydroxyamino) 2(RS)-isobutylsuccinyl]-O-methyl-L-tyrosinylglycinäthylester als Feststoff erhalten. Rf (Chloroform/Methanol 9:1) 0,23.

Beispiel 8

In analoger Weise wie in Beispiel 3 beschrieben wurde [4-(N-Hydroxyamino) 2(RS)-isobutylsuccinyl]-O-methyl-L-tyrosinylglycinäthylamid als Schaum hergestellt. Rf (Chloroform/Methanol 9:1) 0,46.

Beispiel 9

In analoger Weise wie in Beispiel 3 beschrieben wurde [4-(N-Hydroxyamino) 2(RS)-isobutylsuccinyl]-L-leucyl-L-alaninäthylester als Schaum hergestellt. Rf (Chloroform/Methanol 9:1) 0,60 und 0,65.

Beispiel 10

In analoger Weise wie in Beispiel 3 beschrieben wurde [4-(N-Hydroxyamino) 2(RS)-isobutylsuccinyl]-L-leucinpentylamid hergestellt. Rf (Chloroform:Methanol:Essigsäure:Wasser 120:15:3:2) 0,56.

Beispiel 11

In analoger Weise wie in Beispiel 3 beschrieben wurde [4-(N-Hydroxyamino) 2(RS)-isobutylsuccinyl]-L-leucylglycinisopentylester hergestellt. Rf (Aethylacetat) 0,46.

Beispiel 12

In analoger Weise wie in Beispiel 3 beschrieben wurde [4-(N-Hydroxyamino) 2(RS)-isobutylsuccinyl]-L-leucinmethylamid hergestellt. Rf (Chloroform:Methanol:Essigsäure:Wasser 120:15:3:2) 0,45.

Beispiel 13

6,4 g[4-(N-Benzyloxyamino) 2(R)-isobutylsuccinyl]-L-leucyl-L-alaninäthylester wurden in 200 ml Aethanol gelöst und über 1 g 5% Palladium/Kohle bei Raumtemperatur unter Atmosphärendruck hydriert. Nach 1,5 Stunden wurde der Katalysator abfiltriert und das Lösungsmittel abgedampft. Man erhielt 5,0 g [4-N-Hydroxyamino) 2(R)-isobutylsuccinyl]-L-leucyl-L-alaninäthylester als Feststoff vom Schmelzpunkt 175-177 ° C.

Der als Ausgangsmaterial verwendete [4-(N-Benzyloxyamino) 2(R)-isobutylsuccinyl]-L-leucyl-L-alaninäthylester wurde wie folgt hergestellt:

(A) 10,45 g D-Leucinsäure wurden in 150 ml Aethylacetat gelöst. Danach wurden 11 ml Triäthylamin und 18.78 g 4-Nitrobenzylbromid zugesetzt und das Gemissch 3 Stunden zum Rückfluss erhitzt. Das Reaktionsgemisch wurde nacheinander mit Wasser, 10% Zitronensäurelösung, gesättigter Natriumhydrogencarbonatlösung, Wasser und gesättigter Kochsalzlösung gewaschen. Nach Trocknen über Natriumsulfat und Eindampfen wurden 20,6 g D-Leucinsäure-4-nitrobenzylester erhalten. Rf (Chloroform) 0,37.

(B) Eine Lösung von 20,6 g D-Leucinsäure-4-nitrobenzylester und 6,1 g Pyridin in 100 ml trockenem Dichlormethan wurde im Verlauf von 10 Minuten unter Rühren zu einer Lösung von 21,72 g Trifluormethansulfonsäureanhydrid in 180 ml trockenem Dichlormethan bei 0 ° C gegeben. Das Gemisch wurde eine weitere halbe Stunde bei 0 ° C gerührt, mit Wasser und gesättigter Kochsalzlösung gewaschen und über Natriumsulfat getrocknet. Nach Filtration wurde das Filtrat tropfenweise bei 0 ° C zu einer Suspension von 2,3 g 80% Natriumhydrid und 16,63 g di-tert.Butylmalonat in 140 ml trockenem Dimethylformamid gegeben. Das Gemisch wurde 3 Stunden bei Raumtemperatur gerührt, das Lösungsmittel abgedampft und der ölige Rückstand zwischen Aethylacetat und Wasser verteilt. Die wässrige Phase wurde dreimal mit je 50 ml Aethylacetat extrahiert. Die vereinigten Extrakte wurden mit Wasser und gesättigter Kochsalzlösung gewaschen, über Natriumsulfat getrocknet und eingedampft. Man erhielt 34 g 1-(4-nitrobenzyl) 4-tert.butyl 3-tert.butoxycarbonyl 2(R)-isobutylsuccinat als Oel in 95% Ausbeute. Rf (Hexan/Diäthyläther 1:1) 0,38.

(C) 30 g 1-(4-Nitrobenzyl) 4-tert.butyl 3-tert.butoxycarbonyl 2(R)-isobutylsuccinat wurden in 100 ml Trifluoressigsäure gelöst und die Lösung 1 Stunde bei Raumtemperatur gerührt. Das Lösungsmittel wurde abgedampft und der resultierende Gummi mehrere Male mit trockenem Toluol behandelt und das Toluol jeweils abgedampft. Der Rückstand wurde dann in 100 ml trockenem Toluol gelöst und die Lösung 2 Stunden auf 110 ° C erwärmt. Das Lösungsmittel wurde abgedampft und man erhielt 18 g 1-(4-Nitrobenzyl) 2(R)-isobutylhydrogensuccinat als hellgelbes Oel. Rf (Chloroform:Methanol:Essigsäure:Wasser 120:15:3:2) 0,38.

(D) 9,4 g 1-(4-Nitrobenzyl) 2(R)-isobutylhydrogensuccinat wurden in 30 ml trockenem Diäthyläther gelöst und die Lösung in einem Trockeneis/Aceton-Bad gekühlt. Danach wurden 70 ml Isobuten und danach tropfenweise 0,64 ml konzentrierte Schwefelsäure zugesetzt. Das Reaktionsgemisch wurde unter Verschluss 2 Tage bei Raumtemperatur gerührt. Das Gemisch wurde mit gesättigter Natriumhydrogencarbonatlösung alkalisch gemacht und mit Wasser versetzt. Die organische Phase wurde abgetrennt und die wässrige Phase zweimal mit Diäthyläther extrahiert. Die vereinigten ätherischen Phasen wurden mit Wasser und gesättigter Kochsalzlösung gewaschen, über Natriumsulfat getrocknet und eingedampft und lieferten 8,8 g 4-tert.Butyl 1-(4-nitrobenzyl) 2(R)-isobutylsuccinat in Form eines Oels. Rf (Chloroform:Methanol:Essigsäure:Wasser 120:15:3:2) 0,87.

(E) 4,4 g 4-tert.Butyl 1-(4-nitrobenzyl) 2(R)-isobutylsuccinat wurden in 50 ml Aethylactat gelöst und 3,5 Stunden bei Raumtemperatur und Atmosphärendruck über 0,6 g 5% Palladium/Kohle hydriert. Der Katalysator wurde abfiltriert und die Lösung dreimal mit kalter 2N Salzsäure extrahiert. Die organische Phase wurde mit Wasser und gesättigter Kochsalzlösung gewaschen und über Natriumsulfat getrocknet. Das Lösungesmittel wurde abgedampft und das Rohprodukt durch Chromatographie an Silicagel mit Chloroform gereinigt. Man erhielt 2,0 g 4-tert.Butyl 2(R)-isobutylhydrogensuccinat in Form eines Oels. Rf

(Chloroform:Methanol:Essigsäure:Wasser 240:24:3:2) 0,65.

(F) 7,14 g 4-tert.Butyl 2(R)-isobutylhydrogensuccinat und 8,3 g L-Leucyl-L-alaninäthylester-hydrochlorid wurden in 50 ml Dimethylformamid gelöst und in einem Eis/Kochsalz-Bad gekühlt. Danach wurden 4,22 g Hydroxybenzotriazol und 6,43 g N,N′-Dicyclohexylcarbodiimid zugesetzt und anschliessend 4 ml N-Aethylmorpholin. Das Gemisch wurde auf Raumtemperatur aufwärmen gelassen und dann über Nacht gerührt. Der Dicyclohexylharnstoff wurde abfiltriert und das Filtrat zur Trockene eingedampft. Der Rückstand wurde zwischen 150 ml Aethylacetat und 100 ml Wasser verteilt. Die organische Phase wurde nacheinander zweimal mit je 50 ml 5%iger Zitronensäurelösung, zweimal mit je 50 ml gesättigter Natriumhydrogencarbonatlösung, einmal mit 50 ml Wasser und einmal mit 50 ml gesättigter Kochsalzlösung gewaschen und über Natriumsulfat getrocknet. Nach Entfernung des Lösungsmittels durch Eindampfen wurden 15 g eines Feststoffes erhalten, der in 20 ml Aethylacetat suspendiert, mit 50 ml 4N Bromwasserstoff in Essigsäure versetzt wurde, worauf das Gemisch bei Raumtemperatur 50 Minuten gerührt wurde. Das Lösungsmittel wurde unter vermindertem Druck entfernt und der Rückstand mehrmals mit 100 ml Portionen von trockenem Toluol versetzt und eingedampft. Der Rückstand wurde dann in 75 ml Aethylacetat gelöst und dreimal mit je 100 ml gesättigter Natriumhydrogencarbonatlösung extrahiert. Die vereinigten wässrigen Extrakte wurden mit 10% Zitronensäurelösung auf pH 3 angesäuert und das Produkt mit zwei 150 ml-Portionen Aethylacetat extrahiert. Die organische Phase wurde mit Wasser und gesättigter Kochsalzlösung gewaschen und über Natriumsulfat getrocknet. Entfernung des Lösungsmittels unter vermindertem Druck lieferte 8,3 g [2(R)-Isobutylsuccinyl]-L-leucyl-L-alaninäthylesterals Feststoff. Dünnschichtchromatographie zeigte einen grösseren Fleck; Rf (Chloroform/Methanol 19:1) 0,24.

(G) 8,3 g [2(R)-Isobutylsuccinyl]-L-leucyl-L-alaninäthylester wurden in 80 ml trockenem Tetrahydrofuran gelöst und die Lösung auf -15° C gekühlt. Danach wurden 2,75 ml N-Aethylmorpholin zugesetzt und danach 2,81 ml Isobutylchloroformiat. Das Gemisch wurde 5 Minuten bei -15° C gerührt und dann mit 2,65 g O-Benzylhydroxylamin versetzt. Die erhaltene Lösung wurde 30 Minuten bei -15° C und 1 Stunde bei Raumtemperatur gerührt. Das Lösungsmittel wurde abgedampft und der Rückstand zwischen 150 ml Chloroform und 100 ml Wasser verteilt. Die organische Phase wurde nacheinander mit 10% Zitronensäurelösung, Wasser, gesättigter Natriumhydrogencarbonatlösung und gesättigter Kochsalzlösung gewaschen und dann über Natriumsulfat getrocknet. Nach Eindampfen zur Trockene wurde ein Feststoff erhalten, der durch Lösen in heissem Methanol, Zusatz von Aethylacetat und Kühlen auf 0° C umkristallisiert wurde. Man erhielt 6,5 g [4-(N-Benzyloxyamino) 2(R)-isobutylsuccinyl]-L-leucyl-L-alaninäthylester, Schmelzpunkt 179-181° C. Rf (Chloroform/Methanol 19:1) 0,51.

Beispiel 14

0,23 g [4-N-(Benzyloxyamino) 2(R)-propylsuccinyl]-L-leucylglycinäthylester wurden in 10 ml Aethanol 1 Stunde über 0,1 g 5% Palladium/Kohle hydriert. Der Katalysator wurde abfiltriert und das Filtrat zur Trockene eingedampft. Der Rückstand wurde mit Diäthyläther gewaschen und lieferte 0,17 g [4-N-Hyroxyamino) 2(R)-propylsuccinyl]-L-leucylglycinäthylester. Rf (Chloroform:Methanol:Essigsäure:Wasser 120:15:3:2) 0,53.

Der als Ausgangsmaterial verwendete [4-(N-Benzyloxyamino) 2(R)-propylsuccinyl]-L-leucylglycinäthylester wurde wie folgt hergestellt:

(A) 6,3 g D-Norvalinsäure wurden unter Rückfluss in 100 ml Aethyllacetat in Gegenwart von 7,43 ml Triäthylamin und 6,99 ml Benzylbromid 2 Stunden erhitzt. Das Gemisch wurde nacheinander mit Wasser, zweimal 50 ml 10%iger Zitronensäurelösung, Wasser und gesättigter Natriumchloridlösung gewaschen und über Natriumsulfat getrocknet. Das Lösungsmittel wurde abgedampft und das Rohmaterial durch Chromatographie an Silicagel mit 15% Diäthyläther in n-Hexan gereinigt. Man erhielt 6,94 g D-Norvalinsäure-benzylester als Gummi. Rf (Diäthyläther/n-Hexan 1:1) 0,4.

(B) In analoger Weise wie in Beispiel 13(B), 13(C) und 13(D) beschrieben, wurde aus D-Norvalinsäure-benzylester das 4-tert.Butyl 1-benzyl 2(R)-propylsuccinat, Rf (Chloroform:Methanol:Essigsäure:Wasser 120:15:3:2) 0,90 erhalten.

(C) Aus dem 4-tert.Butyl 1-benzyl 2(R)-propylsuccinat wurde die Benzylgruppe durch Hydrierung in Isopropanol in Gegenwart von 5% Palladium/Kohle entfernt. Nach Filtrieren und Eindampfen des Filtrats erhielt man 4-tert.Butyl 2(R)-propylhydrogensuccinat als Oel. Rf (Chloroform:Methanol:Essigsäure:Wasser 120:15:3:2) 0,69.

(D) 0,98 g 4-tert.Butyl 2(R)-propylhydrogensuccinat und 1,14 g L-Leucylglycinäthylester-hydrochlorid wurden in 25 ml trockenem Dimethylformamid gelöst und die Lösung in ein Eis/Kochsalzbad gekühlt. Danach wurden 0,61 g Hydroxybenzotriazol und 0,93 g N,N′-Dicyclohexylcarbodiimid zugesetzt, gefolgt

von 0,58 ml N-Aethylmorpholin. Das Gemisch wurde über Nacht gerührt, der Dicyclohexylharnstoff abfiltriert und das Filtrat zur Trockene eingedampft. Der resultierende Gummi wurde zwischen Aethylacetat und Wasser verteilt. Die organische Phase wurde nacheinander mit 10%iger Zitronensäurelösung, Natriumhydrogencarbonatlösung und gesättigter Kochsalzlösung gewaschen und über Natriumsulfat getrocknet. Das Lösungsmittel wurde abgedampft und das Rohprodukt an Silicagel mit 2% Methanol in Chloroform chromatographiert und lieferte 1,1 g [4-tert.Butyl 2(R)-propylsuccinyl]-L-leucylglycinäthylester. Rf (Chloroform/Methanol 19:1) 0,62.

(E) 1,1 g [4-tert.Butyl 2(R)-propylsuccinyl]-L-leucylglycinäthylester wurden in 7 ml Aethylacetat aufgenommen und die Lösung 1 Stunde mit 14 ml 4N Bromwasserstoff in Essigsäure bei Raumtemperatur gerührt. Das Lösungsmittel wurde abgedampft und der erhaltene Gummi in 15 ml Aethylacetat aufgenommen und dreimal mit je 25 ml gesättigter Natriumhydrogencarbonatlösung extrahiert. Die vereinigten wässrigen Extrakte wurden mit 2N Salzsäure auf etwa pH 3 angesäuert. Die freie Säure wurde dreimal mit je 25 ml Aethylacetat extrahiert, die Extrakte mit Wasser und gesättigter Natriumchloridlösung gewaschen und über Natriumsulfat getrocknet. Das Lösungsmittel wurde abgedampft und man erhielt 0,59 g [2(R)-Propylsuccinyl]-L-leucylglycinäthylester als Oel, das beim Stehenlassen sich verfestigte. Rf (Chloroform/Methanol 9:1) 0,33.

(F) 0,54 g [2(R)-Propylsuccinyl]-L-leucylglycinäthylester wurden mit 0,184 g O-Benzylhydroxylamin nach der gemischten Anhydridmethode wie in Beispiel 13(G) beschrieben, gekuppelt. Nach Aufarbeitung wie in Beispiel 13(G) erhielt man 0,45 g [4-(N-Benzyloxyamino) 2(R)-propylsuccinyl]-L-leucylglycinäthylester als Feststoff. Rf (Chloroform/Methanol 9:1) 0,61.

Beispiel 15

In analoger Weise wie in Beispiel 13 beschrieben, wurde aus 2(RS)-3(S)-Isoleucinsäure der [4-(N-Hydroxyamino) 2(RS)-sek.butylsuccinyl]-L-leucylglycinäthylester erhalten. Rf (Chloroform/Methanol 9:1) 0,40.

Beispiel 16

0,491 g [4-(N-Benzyloxyamino) 2(R)-isobutylsuccinyl]-L-leucyl-L-alaninäthylester in 10 ml Aethanol, 8 ml Wasser und 2 ml 1N Natriumhydroxydlösung wurde 3 Stunden bei Raumtemperatur gerührt. Das Aethanol wurde abgedampft, die Lösung mit 10 ml Wasser verdünnt und zweimal mit je 15 ml Chloroform extrahiert. Die wässrige Lösung wurde mit 2N Salzsäure angesäuert und der erhaltene Feststoff abfiltriert, mit Wasser gewaschen und unter vermindertem Druck getrocknet. Das erhaltene [4-(N-Benzyloxyamino) 2(R)-isobutylsuccinyl]-L-leucyl-L-alanin wurde in 10 ml Aethanol aufgenommen und 2 Stunden über 5% Palladium/Kohle hydriert. Das Gemisch wurde filtriert, das Filtrat eingedampft und das Produkt mit Diäthyläther gewaschen und im Vakuum getrocknet. Man erhielt 0,25 g [4-(N-Hydroxyamino) 2(R)-isobutylsuccinyl]-L-leucyl-L-alanin.

Beispiel 17

0,463 g [4-(N-Benzyloxyamino) 2(RS)-isobutylsuccinyl]-L-leucylglycinmethylester wurden in 5 ml Methanol in Gegenwart von 50 mg 5% Palladium/Kohle 4 Stunden hydriert. Das Gemisch wurde filtriert und das Filtrat eingedampft, wobei 0,335 g [4-Hydroxyamino) 2(RS)-isobutylsuccinyl]-L-leucylglycinmethylester als Schaum erhalten wurden.

Der als Ausgangsmaterial verwendete [4-(N-Benzyloxyamino) 2(RS)-isobutylsuccinyl]-L-leucylglycinmethylester wurde wie folgt hergestellt:

(A) 45,2 g 4-tert.Butyl 2(RS)-isobutylhydrogensuccinat in 150 ml Dichlormethan wurden bei 0 °C mit einer auf 0 °C gekühlten Lösung von 42,8 g Methyl L-leucinat-hydrochlorid und 30 ml N-Aethylmorpholin in 200 ml Dichlormethan versetzt. Danach wurden 31,8 g Hydroxybenzotriazol und dann 44,4 g N,N'-Dicyclohexylcarbodiimid in 110 ml Dichlormethan zugesetzt. Das Gemisch wurde 1 Stunde bei 0 °C gerührt, über Nacht bei 4 °C stehengelassen, filtriert und eingedampft. Der Rückstand wurde in Aethylacetat aufgenommen und nacheinander mit Wasser, 5%iger Zitronensäurelösung, Wasser, 5% Natriumhydrogencarbonatlösung und gesättigter Kochsalzlösung gewaschen, über Magnesiumsulfat getrocknet und eingedampft. Man erhielt 71 g [4-tert.Butyl 2(RS)-isobutylsuccinyl]-L-leucinmethylester als Gel. Rf (n-Hexan/Aethylacetat 2:1) 0,87.

(B) 64 g [4-tert.Butyl 2(RS)-isobutylsuccinyl]-L-leucinmethylester und 130 ml Aethylacetat wurden eine halbe Stunde mit 100 ml 4N Bromwasserstoff in Essigsäure behandelt. Das Lösungsmittel wurde

abgedampft und letzte Spuren von Essigsäure durch dreimaliges Abdampfen mit je 100 ml Toluol entfernt. Der Rückstand wurde in 150 ml Aethylacetat aufgenommen und dreimal mit je 150 ml 5% Natriumhydrogencarbonatlösung extrahiert. Die wässrigen Lösungen wurden vereinigt, mit 2N Salzsäure angesäuert und zweimal mit je 200 ml Aethylacetat extrahiert. Die Lösung wurde zweimal mit je 100 ml gesättigter Kochsalzlösung gewaschen, über Magnesiumsulfat getrocknet und eingedampft und lieferte 37,1 g [2(RS)-Isobutylsuccinyl]-L-leucinmethylester als Oel. Rf (Chloromethan:Methan:Essigsäure:Wasser 120:15:3.2) 0,58.

(C) Eine Lösung von 37,1 g [2(RS)-Isobutylsuccinyl]-L-leucinmethylester in 200 ml Tetrahydrofuran wurde bei -12°C mit 15,65 ml N-Aethylmorpholin und 16,14 ml Isobutylchloroformiat versetzt. Das Gemisch wurde 4 Minuten bei -12°C gerührt, mit 15,2 g O-Benzylhydroxylamin versetzt, 1 Stunde bei 0°C gerührt, 64 Stunden bei 0°C stehengelassen und dann eingedampft. Der Rückstand wurde in 200 ml Aethylacetat aufgenommen und nacheinander mit Wasser, 5% Zitronensäurelösung, Wasser und 5%iger Natriumhydrogencarbonatlösung gewaschen, über Magnesiumsulfat getrocknet und zu einem Oel eingedampft. Chromatographie an 500 g Silicagel mit Methanol/Dichlormethan (1:24) lieferte 35,5 g [4-(N-Benzyloxyamino) 2(RS)-isobutylsuccinyl]-L-leucinmethylester als Isomergengemisch. Rf (Chloroform/Methanol 9:1) 0,30, 0,46.

(D) 35,5 g [4-(N-Benzyloxyamino) 2(RS)-isobutylsuccinyl]-L-leucinmethylester in 200 ml Methanol und 180 ml Wasser wurden 16 Stunden bei Raumtemperatur mit 22 ml 4N Natriumhydroxydlösung gerührt. Die Suspension wurde zur Entfernung des Methanols eingedampft, mit 200 ml Wasser versetzt und mit Aethylacetat extrahiert. Die wässrige Lösung wurde mit 2N Salzsäure angesäuert. Das Produkt wurde in Aethylacetat aufgenommen, mit Wasser und gesättigter Kochsalzlösung gewaschen, über Magnesiumsulfat getrocknet und eingedampft. Man erhielt 17,5 g [4-(N-Benzyloxyamino) 2(RS)-isobutylsuccinyl]-L-leucinals Schaum.

Die erste Aethylacetatlösung wurde mit Wasser und gesättigter Kochsalzlösung gewaschen, über Magnesiumsulfat getrocknet und eingedampft. Man erhielt [4-(N-Benzyloxyamino) 2(RS)-isobutylsuccinyl]-L-leucinmethylester, der in 200 ml methanol und 180 ml Wasser 4 Stunden mit 22 ml 4N Natriumhydroxydlösung gerührt wurde. Nach Aufarbeitung, wie im vorstehenden Absatz beschrieben, erhielt man weitere 13,2 g [4-(N-Benzyloxyamino) 2(RS)-isobutylsuccinyl]-L-leucin. Die Gesamtausbeute betrug 30,7 g.

(E) 2,07 g [4-(N-Benzyloxyamino) 2(RS)-isobutylsuccinyl]-L-leucin in 20 ml Tetrahydrofuran wurden bei 0°C mit einer auf 0°C gekühlten Lösung von 0,63 g Methylglycinat-hydrochlorid und 0,64 ml N-Aethylmorpholin in 10 ml Chloroform versetzt. Danach wurden 0,81 g Hydroxybenzotriazol und 1,13 g N,N′-Dicyclohexylcarbodiimid zugesetzt. Das Gemisch wurde 1 Stunde bei 0°C gerührt, 16 Stunden bei 0°C stehengelassen, filtriert und eingedampft. Der Rückstand wurde in Aethylacetat aufgenommen und nacheinander mit 5% Zitronensäurelösung, Wasser, 5% Natriumhydrogencarbonatlösung und gesättigter Kochsalzlösung gewaschen, über Magnesiumsulfat getrocknet und eingedampft. Chromatrographie des Rückstandes an 25 g Silicagel mit Aethylacetat/Hexan (1:1) lieferte 1,9 g [4-(N-Benzyloxyamino) 2(RS)-isobutylsuccinyl]-L-leucylglycinmethylester als Schaum.

## Beispiel 18

In analoger Weise wie in Beispiel 17 beschrieben, wurden die folgenden Verbindungen hergestellt:

[4-(N-Hydroxyamino 2(RS)-isobutylsuccinyl]-L-leucylsarcosinäthylester;
[4-(N-Hydroxyamino) 2(RS)-isobutylsuccinyl]-L-leucyl-L-prolinäthylester;
[4-(N-Hydroxyamino) 2(RS)-isobutylsuccinyl]-L-leucyl-L-alaninisopropylester;
[4-(N-Hydroxyamino) 2(RS)-isobutylsuccinyl]-L-leucin-isopropylamid;
[4-(N-Hydroxyamino) 2(RS)-isobutylsuccinyl]-L-leucin-2-oxopropylamid;
[4-N-Hydroxyamino) 2(RS)-isobutylsuccinyl]-L-leucin-2-methoxyäthylamid;
[4-(N-Hydroxyamino) 2(RS)-isobutylsuccinyl]-L-leucin-2,2-dimethoxyäthylamid.

## Beispiel 19

0,4 g [N-Formyl-N-benzyloxy 2(RS)-isobutyl-3-aminopropionyl]-L-leucylglycinäthylester in 20 ml Aethanol wurden 0,75 Stunden in Gegenwart von 5% Palladium/Kohle hydriert. Das Gemisch wurde filtriert, das Filtrat eingedampft und der Rückstand unter vermindertem Druck getrocknet. Man erhielt 0,32 g [N-Formyl-N-hydroxy 2(RS)-isobutyl-3-aminopropionyl]-L-leucylglycinäthylester als Schaum.

Der als Ausgangsmaterial verwendete [N-Formyl-N-benzyloxy 2(RS)-isobutyl-3-aminopropionyl]-L-leucylglycinäthylester wurde wie folgt hergestellt:

(A) 2,85 g Isobutylacrylsäure und 10,9 g O-Benzylhydroxylamin wurden in 30 ml Methanol 8 Tage zum Rückfluss erhitzt. Das Lösungsmittel wurde abgedampft und der Rückstand in Aethylacetat und Wasser gelöst. Die organische Phase wurde mit 2N Salzsäure und Wasser gewaschen, dann sechsmal mit je 30 ml 5% Natriumhydrogencarbonatlösung gewaschen. Die wässrigen Lösungen wurden vereinigt, mit 2N Salzsäure angesäuert und mit Aethylacetat extrahiert. Der Aethylacetatextrakt wurde mit Wasser und gesättigter Kochsalzlösung gewaschen, über Magnesiumsulfat getrocknet und zu einem Oel, das bald kristallisierte, eingedampft. Verreiben mit Petroläther lieferte 1,92 g N-Benzyloxy 2(RS)-isobutyl-3-aminopropionsäure, Schmelzpunkt 73-75 °C. Rf (Toluol/Essigsäure 4:1) 0,52.

(B) 1 g N-Benzyloxy 2(RS)-isobutyl-3-aminopropionsäure in 10 ml Ameisensäure und 1 ml Essigsäureanhydrid wurden 2 Stunden bei Raumtemperatur gerührt und dann eingedampft. Der Rückstand wurde in Aethylacetat aufgenommen, mit Wasser und gesättigter Kochsalzlösung gewaschen, über Magnesiumsulfat getrocknet und eingedampft. Man erhielt 1,2 g N-Formyl-N-benzyloxy 2(RS)-isobutyl-3-aminopropionsäure als Gummi. Rf (Toluol/Essigsäure 4:1) 0,39.

(C) In analoger Weise wie in Beispiel 1(D) beschrieben, wurden aus 1,2 g N-formyl-N-benzyloxy 2(RS)-isobutyl-3-aminopropionsäure 1,17 g [N-formyl-N-benzyloxy 2(RS)-isobutyl-3-aminopropionyl]-L-leucylglycinäthylester als Gemisch eines Oels und von Kristallen erhalten. Rf (Aethylacetat/n-Hexan 7:3) 0,51.

Beispiel 20

0,12 g $N^{\alpha}$-[4-(N-Benzyloxyamino) 2(R)-isobutylsuccinyl]-$N^{\epsilon}$-(N-benzyloxycarbonylglycyl) -L-lysyl-L-alaninäthylester wurden in 20 ml Aethanol in Gegenwart von 0,1 g 5% Palladium/Kohle und 0,172 ml 1N Salzsäure 1 Stunde hydriert. Der Katalysator wurde abfiltriert und das Lösungsmittel abgedampft. Man erhielt 70 mg $N^{\alpha}$-[4-(N-Hydroxyamino) 2(R)-isobutylsuccinyl]-$N^{\epsilon}$-glycyl-L-lysyl-L-alaninäthylester-hydrochlorid als Feststoff. Rf (Chloroform:Methanol:Essigsäure:Wasser 60:18:2:3) 0,3.

Der als Ausgangsmaterial verwendete $N^{\alpha}$-[4-(N-Benzyloxyamino) 2(R)-isobutylsuccinyl]-$N^{\epsilon}$-(N-benzyloxycarbonylglycyl) -L-lysyl-L-alaninäthylester wurde wie folgt hergestellt:

(A) 13,3 g $N^{\alpha}$-tert.Butoxycarbonyl-$N^{\epsilon}$-benzyloxycarbonyl-L-lysin wurden in 100 ml Tetrahydrofuran gelöst und die Lösung auf -20 °C gekühlt. Danach wurden 4,58 ml Isobutylchloroformiat und 4,48 ml N-Aethylmorpholin zugesetzt. Nach 2 Minuten Rühren bei -20 °C wurden 5,37 g L-Alaninäthylester-hydrochlorid und 4,48 ml N-Aethylmorpholin zugesetzt und das Gemisch auf Raumtemperatur aufwärmen gelassen, worauf weitere 4 Stunden gerührt wurde. Das Lösungsmittel wurde abgedampft und der Rückstand zwischen 200 ml Aethylacetat und 100 ml Wasser verteilt. Die organische Phase wurde nacheinander zweimal mit je 25 ml 10%iger Zitronensäurelösung, zweimal mit je 25 ml gesättigter Kochsalzlösung und einmal mit 25 ml Wasser und einmal mit 25 ml gesättigter Kochsalzlösung gewaschen, getrocknet und eingedampft. Der erhaltene Gummi kristallisierte aus Aethylacetat/n-Hexan. Man erhielt 13 g $N^{\alpha}$-tert.Butoxycarbonyl-$N^{\epsilon}$]benzyloxycarbonyl-L-lysyl -L-alaninäthylester, Rf (Chloroform/Methanol 9:1) 0,73.

(B) 13 g $N^{\alpha}$-tert.Butoxycarbonyl-$N^{\epsilon}$]benzyloxycarbonyl-L-lysyl -L-alaninäthylester wurden in 15 ml 4N Chlorwasserstoff in Aethylacetat gelöst und die Lösung bei Raumtemperature 0,5 Stunden gerührt. Das Lösungsmittel wurde unter vermindertem Druck entfernt und das Produkt im Hochvakuum getrocknet. Man erhielt 11,5 g $N^{\epsilon}$-Benzyloxycarbonyl-L-lysyl -L-alaninäthylester-hydrochlorid als Feststoff.

(C) 4,5 g 4-tert.Butyl 2(R)-isobutylhydrogensuccinat wurden in einem Gemisch von 20 ml Dimethylformamid und 20 ml Tetrahydrofuran gelöst. Die Lösung wurde auf -20 °C gekühlt und mit 2,5 ml N-Aethylmorpholin und 2,56 ml Isobutylchloroformiat versetzt. Nach 2 Minuten wurden 8,13 g $N^{\epsilon}$-Benzyloxycarbonyl-L-lysyl -L-alaninäthylester-hydrochlorid und danach 2,5 ml N-Aethylmorpholin zugesetzt. Das Gemisch wurde 4 Stunden bei Raumtemperatur gerührt und eingedampft. Der Rückstand wurde in Aethylacetat aufgenommen, nacheinander mit 10% Essigsäurelösung, Wasser, 5% Natriumhydrogencarbonatlösung und gesättigter Kochsalzlösung gewaschen, über Natriumsulfat getrocknet und eingedampft. Man erhielt 13 g Rohprodukt, das durch Chromatographie an Silicagel mit Chloroform als Elutionsmittel gereinigt wurde. Man erhielt 9,8 g reines $N^{\alpha}$-[4-tert.Butyl 2(R)-isobutylsuccinyl]-$N^{\epsilon}$-benzyloxycarbonyl-L-lysyl -L-alaninäthylester als hellgelbes Oel. Rf (chloroform/Methanol 9:1) 0,54.

(D) 5,65 g $N^{\alpha}$-[4-tert.Butyl 2(R)-isobutylsuccinyl]-$N^{\epsilon}$-benzyloxycarbonyl-L-lysyl -L-alaninäthylester wurden in 200 ml Aethanol in Gegenwart von 500 mg 5% Palladium/Kohle und 9,56 ml 1N Salzsäure 3 Stunden hydriert. Der Katalysator wurde abfiltriert und das Filtrat unter vermindertem Druck eingedampft. Man erhielt 4,7 g $N^{\alpha}$-[4-tert.Butyl 2(R)-isobutylsuccinyl]-L-lysyl -L-alaninäthylester-hydrochlorid als Gummi.

(E) 2,25 g des vorstehenden Hydrochlorids und 0,953 g N-Benzyloxycarbonylglycin wurden in 5 ml Dimethylformamid gelöst und die Lösung in einem Eis/Kochsalzbad gekühlt. Danach wurden 0,62 g Hydroxybenzotriazol und 0,94 g N,N'-Dicyclohexylcarbodiimid zugesetzt, gefolgt von 0,58 ml N-Aethylmorpholin. Das Gemisch wurde über Nacht bei Raumtemperatur gerührt und der Dicyclohexylharnstoff abfiltriert. Danach wurde das Lösungsmittel abgedampft und der erhaltene Gummi zwischen Aethylacetat und Wasser verteilt. Die organische Phase wurde nacheinander mit 10% Zitronensäurelösung, gesättigter Natriumhydrogencarbonatlösung, Wasser und gesättigter Kochsalzlösung gewaschen und über Natriumsulfat getrocknet. Das Lösungsmittel wurde unter vermindertem Druck entfernt, und das Rohprodukt durch Chromatographie an Silicagel mit Chloroform und 2% Methanol in Chloroform gereinigt. Man erhielt 1,5 g reines $N^{\alpha}$-[4-tert.Butyl 2(R)-isobutylsuccinyl]-$N^{\epsilon}$-(N-benzyloxycarbonylglycyl) -L-lysyl-L-alaninäthylester. Rf (Chloroform:Methanol:Essigsäure:Wasser 120:15:3:2) 0,67.

(F) 1,5 g $N^{\alpha}$-[4-tert.Butyl 2(R)-isobutylsuccinyl]-$N^{\epsilon}$-(N-benzyloxycarbonylglycyl) -L-lysyl-L-alaninäthylester wurden in 10 ml Trifluoressigsäure gelöst und die Lösung 1 Stunde bei Raumtemperatur gerührt. Das Lösungsmittel wurde unter vermindertem Druck abgedampft und das Produkt 5 Stunden über Kaliumhydroxyd im Hochvakuum getrocknet. Man erhielt 1,24 g $N^{\alpha}$-[2(R)-Isobutylsuccinyl]-$N^{\epsilon}$-(N-benzyloxycarbonylglycyl) -L-lysyl-L-alaninäthylester als weissen Feststoff.

(G) 1,24 g $N^{\alpha}$-[2(R)-Isobutylsuccinyl]-$N^{\epsilon}$-(N-benzyloxycarbonylglycyl) -L-lysyl-L-alaninäthylester und 0.26 g O-Benzylhydroxylamin wurden in 15 ml Dimethylformamid mit 0,283 g Hydroxybenzotriazol und 0,43 g Dicyclohexylcarbodiimid in Gegenwart von 0,24 g N-Aethylmorpholin gekuppelt. Nach Rühren über Nacht wurde das Gemisch, wie in Absatz (E) oben beschrieben, aufgearbeitet. Das Rohprodukt wurde durch Chromatographie an Silicagel mit Chloroform und 2% Methanol in Chloroform gereinigt. Man erhielt 0,78 g $N^{\alpha}$-[4-(N-Benzyloxyamino) 2(R)-isobutylsuccinyl]-$N^{\epsilon}$-(N-benzyloxycarbonylglycyl) -L-lysyl-L-alaninäthylester als weissen Feststoff. Rf (Chloroform/Methanol 19:1) 0,49; (Chloroform:Methanol:Essigsäure:Wasser 120:15:3:2) 0,68.

## Beispiel 21

In analoger Weise wie in Beispiel 20 beschrieben, wurde $N^{\alpha}$-[4-(N-Hydroxyamino) 2(R)-isobutylsuccinyl]-$N^{\epsilon}$-glycyl-L-lysinmethylamid hergestellt. Rf (Chloroform:Methanol:Essigsäure:Wasser 60:18:2:3) 0,19.

## Beispiel 22

0,15 g $N^{\alpha}$-[4-(N-Benzyloxyamino) 2(R)-isobutylsuccinyl]-$N^{\epsilon}$-(4-benzyloxycarbonylbenzoyl) -L-lysyl-L-alaninäthylester wurden in 15 ml Dimethylformamid gelöst und in Gegenwart von 0,1 g 5% Palladium/Kohle 1 Stunde hydriert. Der Katalysator wurde abfiltriert und das Lösungsmittel unter vermindertem Druck entfernt. Der Rückstand wurde mit Diäthyläther verrieben, abfiltriert und unter vermindertem Druck getrocknet. Man erhielt 80 mg $N^{\alpha}$-[4-(N-Hydroxyamino) 2(R)-isobutylsuccinyl]-$N^{\epsilon}$-(4-carboxybenzoyl) -L-lysyl-L-alaninäthylester als Feststoff, Rf (Chloroform:Methanol:Essigsäure:Wasser 60:18:2:3) 0,79.

Der als Ausgangsmaterial verwendete $N^{\alpha}$-[4-(N-Benzyloxyamino) 2(R)-isobutylsuccinyl]-$N^{\epsilon}$-(4-benzyloxycarbonylbenzoyl) -L-lysyl-L-alaninäthylester wurde wie folgt hergestellt:

(A) $N^{\alpha}$-[4-tert.Butyl 2(R)-isobutylsuccinyl]-L-lysyl -L-alaninäthylester-hydrochlorid und 4-Benzyloxycarbonylbenzoesäure wurden in der in Beispel 20(E) beschriebenen Weise gekuppelt. Das nach Aufarbeitung erhaltene Rohprodukt wurde durch Chromatographie an Silicagel durch Gradientelution mit Chloroform, das bis zu 5% Methanol enthielt, gereinigt. Man erhielt den $N^{\alpha}$-[4-tert.Butyl 2(R)-isobutylsuccinyl]-$N^{\epsilon}$(4-benzyloxycarbonylbenzoyl)-L-lysyl-L-alaninäthylester als gelbes Oel. Rf (Chloroform/Methanol 9:1) 0,71.

(B) 2,9 g $N^{\alpha}$-[4-tert.Butyl 2(R)-isobutylsuccinyl]-$N^{\epsilon}$(4-benzyloxycarbonylbenzoyl)-L-lysyl -L-alaninäthylester wurden in 20 ml Trifluoressigsäure gelöst und die Lösung 20 Minuten bei Raumtemperatur gerührt. Das Lösungsmittel wurde abgedampft und das Rohprodukt durch Chromatographie an Silicagel mit 5% Methanol in Chloroform gereinigt. Man erhielt 0,735 g $N^{\alpha}$-[2(R)-Isobutylsuccinyl]-$N^{\epsilon}$-(4-benzyloxycarbonylbenzoyl)-L-lysyl-L-alaninäthylester. Dieser wurde mit O-Benzylhydroxylamin unter Verwendung von Dicyclohexylcarbodiimid/Hydroxybenzotriazol in Analogie zu dem Verfahren von Beispiel 20(G) gekuppelt. Nach Aufarbeitung wurde das Rohprodukt durch Chromatographie an Silicagel mit Gradientelution mittels Chloroform, das bis zu 5% Methanol enthielt, gereinigt. Man erhielt 0,6 g $N^{\alpha}$-[4-(N-Benzyloxyamino) 2(R)-isobutylsuccinyl]-$N^{\epsilon}$-(4-benzyloxycarbonylbenzoyl) -L-lysyl-L-alaninäthylester als weissen Feststoff. Rf (Chloroform/Methanol 19:1) 0,37.

## Beispiel 23

0,16 g $N^{\alpha}$-[4-(N-Benzyloxyamino) 2(R)-isobutylsuccinyl]-$N^{\epsilon}$-(4-carboxybenzoyl) -L-lysyl-L-alanin wurden in 5-10 ml Dimethylformamid gelöst und in Gegenwart von 0,1 g 5% Palladium/Kohle 1 Stunde hydriert. Der

Katalysator wurde abfiltriert und das Filtrat eingedampft. Der Feststoff wurde mit Diäthyläther verrieben, abfiltriert und unter vermindertem Druck getrocknet. Man erhielt 0,1 g $N^{\alpha}$-[4-(N-Hydroxyamino) 2(R)-isobutylsuccinyl]-$N^{\epsilon}$-(4-carboxybenzoyl) -L-lysyl-L-alanin als Feststoff. Rf (Chloroform:Methanol:Essigsäure:Wasser 60:18:2:3) 0,78.

Das als Ausgangsmaterial verwendete $N^{\alpha}$-[4-(N-Benzyloxyamino) 2(R)-isobutylsuccinyl]-$N^{\epsilon}$-(4-carboxybenzoyl) -L-lysyl-L-alanin wurde wie folgt hergestellt:

0,2 g $N^{\alpha}$-[4-(N-Benzyloxyamino) 2(R)-isobutylsuccinyl]-$N^{\epsilon}$-(4-benzyloxycarbonylbenzoyl) -L-lysyl-L-alaninäthylester wurden in 5 ml Dimethylformamid gelöst und in Gegenwart von 0,55 ml 1N Natriumhydroxydlösung bei Raumtemperatur gerührt. Nach 2 Stunden wurde das Lösungsmittel abgedampft und der Rückstand in Wasser aufgenommen und auf ein pH von etwa 3 mit 1N Salzsäure angesäuert. Das Produkt wurde abfiltriert, mit Wasser und Diäthyläther gewaschen und unter vermindertem Druck getrocknet. Man erhielt 0,16 g $N^{\alpha}$-[4-(N-Benzyloxyamino) 2(R)-isobutylsuccinyl]-$N^{\epsilon}$-(4-carboxybenzoyl) -L-lysyl-L-alanin, Rf (Chloroform:Methanol:Essigsäure:Wasser 60:18:2:3) 0,85.

## Beispiel 24

0,35 g [4-(N-Benzyloxyamino) 2(R)-isobutylsuccinyl]-3(RS)-aminolaurolactam wurden in 15 ml Methanol suspendiert und 2 Stunden über 5% Palladium/Kohle hydriert. Das Gemisch wurde abfiltriert, das Filtrat eingedampft und der Rückstand mit Diäthyläther gewaschen. Man erhielt 0,27 g [4-(N-Hydroxyamino) 2(R)-isobutylsuccinyl]-3(RS)-aminolaurolactam als Feststoff vom Schmelzpunkt 200-205 ° C.

Das Ausgangsmaterial wurde wie folgt hergestellt:

(A) 19,7 g Laurolactam in 100 ml Chloroform wurden auf 0 ° C gekühlt und die Lösung im Verlauf von 40 Minuten mit 20,8 g Phosphorpentachlorid portionenweise versetzt, wobei die Temperatur unterhalb 5 ° C gehalten wurde. Darauf wurden 0,2 g Jod, gefolgt von 16 g Brom im Verlauf von 20 Minuten zugesetzt. Das Gemisch wurde 4 Stunden zum Rückfluss erhitzt, gekühlt, eingedampft und auf Eis gegossen. Das Produkt wurde in Chloroform aufgenommen, mit Natriumhydrogensulfitlösung gewaschen, über Magnesiumsulfat getrocknet und eingedampft. Kristallisation aus Chloroform/n-Hexan lieferte 12,65 g 3-Bromlaurolactam vom Schmelzpunkt 155-157 ° C.

(B) 9,3 g 3-Bromlaurolactam und 8,6 g Natriumazid in 100 ml Dimethylformamid wurden 64 Stunden auf 80 ° C erwärmt und dann eingedampft. Der Rückstand wurde in Chloroform aufgenommen, mit Wasser gewaschen, über Magnesiumsulfat getrocknet und eingedampft, wobei das Azid in Form eines Feststoffes erhalten wurde. Dieser Feststoff wurde in 70 ml Aethanol aufgenommen und 64 Stunden über 5% Palladium/Kohle hydriert. Die Lösung wurde filtriert und eingedampft und der Rückstand in Chloroform gelöst. Das Produkt wurde in Wasser, das 17 ml 2N Salzsäure enthielt, extrahiert. Eindampfe, Trocknen und Kristallisation aus Aethanol/Diäthyläther lieferte 4,9 g 3-Aminolaurolactam-hydrochlorid als weissen Feststoff vom Schmelzpunkt 251-254 ° C.

(C) 1,73 g 3-Aminolaurolactam-hydrochlorid in 20 ml Dimethylformamid wurden bei 0 ° C mit 0,88 ml N-Aethylmorpholin versetzt. Danach wurden 1,6 g 4-tert.Butyl 2(R)-isobutylhydrogensuccinat, 1,14 g Hydroxybenzotriazol und 1,59 g N,N′-Dicyclohexylcarbodiimid zugesetzt, das Gemisch 1 Stunde bei 0 ° C gerührt und dann über Nacht bei 0 ° C stehengelassen. Die Lösung wurde dann filtriert, das Filtrat eingedampft und der Rückstand in Aethylacetat gelöst, die Lösung nacheinander mit 5% Zitronensäurelösung, Wasser, 5% Natriumhydrogencarbonatlösung und gesättigter Kochsalzlösung gewaschen, über Magnesiumsulfat getrocknet und zu einem Oel eingedampft. Das erhaltene rohe [4-tert.Butyl 2(R)-isobutylsuccinyl]-3(RS)-aminolaurolactam wurde in 10 ml Aethylacetat eine halbe Stunde mit 10 ml 4N Bromwasserstoff in Essigsäure behandelt. Das Gemisch wurde dann eingedampft, dreimal mit 20 ml-Portionen Toluol versetzt und erneut bei Entfernung von Spuren von Essigsäure eingedampft. Der Rückstand, ein Schaum, wurde in 20 ml Tetrahydrofuran gelöst und auf -12 ° C gekühlt. Danach wurden 1,11 ml N-Aethylmorpholin und 0,84 ml Isobutylchloroformiat zugesetzt und das Gemisch 4 Minuten bei -12 ° C gerührt. Darauf wurden 0,94 g O-Benzylhydroxylamin zugesetzt, das Gemisch 1 Stunde bei 0 ° C und 1 Stunde bei Raumtemperatur gerührt und dann zu einem Feststoff eingedampft. Das Produkt wurde mit Wasser und Diäthyläther gewaschen und aus Chloroform/Diäthyläther umkristallaisiert. Man erhielt 1,4 g [4-(N-Benzyloxyamino) 2(R)-isobutylsuccinyl]-3(RS)-aminolaurolactam als weissen Feststoff vom Schmelzpunkt 215-222 ° C.

## Beispiel 25

In analoger Weise wie in Beispiel 24 beschrieben, wurde das [4-(N-Hydroxyamino) 2(R)-isobutylsuccinyl]-3(RS)-amino-octahydro-2H-azonin-2-on als Schaum hergestellt. Rf

(Chloroform:Methanol:Essigsäure:Wasser 120:15:3:2) 0,44.

Beispiel 26

0,4 [4-(N-Benzyloxyamino) 3(S)-methyl-2(R)-isobutylsuccinyl]-L-leucylglycinäthylester wurden in 10 ml Aethanol 2 Stunden über 5% Palladium/Kohle hydriert. Das Gemisch wurde filtriert, das Filtrat eingedampft, der Rückstand mit Dichlormethan versetzt und erneut eingedampft. Man erhielt 0,28 g [4-(N-Hydroxyamino) 3(S)-methyl-2(R)-isobutylsuccinyl]-L-leucylglycinäthylester in Form eines rosa Feststoffs, Rf (Chloroform:Methanol:Essigsäure:Wasser 120.15:3:2) 0,56.

Das Ausgangsmaterial wurde wie folgt hergestellt:

(A) 1-Benzyl 4-tert.butyl 3 tert.butyloxycarbonyl-2(S)-methylsuccinat wurde aus Benzyl S-lactat in analoger Weise wie in Beispiel 13(B) beschrieben, hergestellt. Dieses Material enthielt 13% di-tert.Butylmalonat.

(B) 11,77 g rohes 1-Benzyl 4-tert.butyl 3-tert.butyloxycarbonyl-2(S)-methylsuccinat in 100 ml Dimethylformamid wurde mit 1,01 g 80% Natriumhydrid behandelt. Nach 0,25 Stunden wurden 3,86 ml Isobutyljodid zugesetzt und das Gemisch 2 Stunden auf 80 °C erwärmt. Danach wurde eine gleiche Menge der Reagentien zugesetzt und das Erwärmen auf 80 °C weitere 2 Stunden fortgesetzt. Das Gemisch wurde gekühlt und eingedampft und der Rückstand in Aethylacetat gelöst. Die Aethylacetatlösung wurde mit Wasser und gesättigter Kochsalzlösung gewaschen, über Magnesiumsulfat getrocknet und zu einem gelben Oel eingedampft. Chromatographie an 250 g Silicagel mit Aethylacetat/n-Hexan (1:15) lieferte 8,76 g 1-Benzyl 4-tert.butyl 3-tert.butyloxycarbonyl 3-isobutyl-2(S)-methylsuccinat als Oel. Rf (Aethylacetat/n-Hexan 1:9) 0.70.

(C) 8,76 g 1-Benzyl 4-tert.butyl 3-tert.butxyloxycarbonyl-3-isobutyl-2(S)-metrhylsuccinat wurden in 10 ml Trifluoressigsäure 1 Stunde bei Raumtemperatur gerührt. Danach wurde eingedampft, Spuren von Trifluoressigsäure wurden durch Zusatz von drei 20 ml-Portionen Toluol und Abdampfen entfernt. Der Rückstand wurde in Toluol aufgenommen, die Lösung 3 Stunden zum Rückfluss erhitzt und dann zu einem Oel eingedampft. Das Oel wurde in Aethylacetat aufgenommen, die Lösung dreimal mit je 50 ml Natriumhydrogencarbonat extrahiert, die wässrige Phase mit 2N Salzsäure angesäuert und mit Aethylacetat extrahiert. Die organische Phase wurde mit Wasser und gesättigter Kochsalzlösung gewaschen, über Magnesiumsulfat getrocknet und eingedampft. Man erhielt 2,55 g 4-Benzyl 2(R)-isobutyl-3(S)-methylhydrogensuccinat als Oel. Rf (Chloroform:Methanol:Essigsäure:Wasser 120:15:3:2) 0,60.

(D) 1,76 g 4-Benzyl 2(R)-isobutyl-3(S)-methylhydrogensuccinat wurden mit L-Leucylglycinäthylester-hydrobromid nach der Hydroxybenzotriazol/Dicyclohexylcarbodiimid-Methode, die in Beispiel 1(D) beschrieben ist, gekuppelt. Chromatographie an 60 g Silicagel mit Aethylacetat/n-Hexan (4:6) lieferte 1,4 g [4-Benzyl 3(S)-methyl-2(R)-isobutylsuccinyl]-L-leucylglycinäthylester als Oel. Rf (Aethylacetat/n-Hexan 1:1) 0,53.

1,32 g [4-Benzyl 3(S)-methyl-2(R)-isobutylsuccinyl]-L-leucylglycinäthylester wurden in 20 ml Dimethylformamid 4 Stunden in Gegenwart von 0,15 g 5% Palladium/Kohle hydriert. Das Gemisch wurde filtriert, das Filtrat auf -12 °C gekühlt, mit 0,35 ml N-Aethylmorpholin und 0,36 ml Isobutylchloroformat versetzt und 4 Minuten bei -12 °C gerührt. Danach wurden 0,34 g O-Benzylhydroxylamin zugesetzt, das Gemisch 2 Stunden bei 0 °C gerührt und das Lösungsmittel abgedampft. Der Rückstand wurde in Chloroform aufgenommen, nacheinander mit 5% Zitronensäurelösung, Wasser, 5% Natriumhydrogencarbonatlösung und gesättigter Kochsalzlösung gewaschen, über Magnesiumsulfat getrocknet und zu einem Feststoff eingedampft. Chromatographie an 30 g Silicagel mit Chloroform lieferte 0,45 g [4-(N-Benzyloxyamino) 3(S)-methyl-2(R)-isobutylsuccinyl]-L-leucylglycinäthylester als Feststoff.

Beispiel 27

In analoger Weise wie in Beispiel 26 beschrieben, wurde aus L-Lysin der [4-(N-Hydroxyamino) 3(S)-phthaloylaminobutyl-2(R)-isobutylsuccinyl] -L-leucylglycinäthylester als Feststoff vom Schmelzpunkt 197 °C (Zersetzung) erhalten. Rf (Chloroform:Methanol:Essigsäure:Wasser 120:15:3:2) 0,64.

Beispiel 28

In analoger Weise wie in Beispiel 19 beschrieben, wurden der [N-Formyl-N-hydroxy 2(RS)-isobutyl-3-aminopropionyl]-L-leucyl-L-alaninäthylester und das [N-Formyl-N-hydroxy 2(RS)-isobutyl-3-aminopropionyl]-L-leucinmethylamid als Schäume hergestellt.

Beispiel A

Tabletten mit den folgenden Inhaltsstoffen können in üblicher Weise hergestellt werden:

| Inhaltsstoff | | pro Tablette |
|---|---|---|
| Hydroxylaminderivat | | 10,0 mg |
| Lactose | | 125,0 mg |
| Maisstärke | | 75,0 mg |
| Talkum | | 4,0 mg |
| Magnesiumstearat | | 1,0 mg |
| | Totalgewicht | 215,0 mg |

Beispiel B

Kapseln mit den folgenden Inhaltsstoffen können in üblicher Weise hergestellt werden:

| Inhaltsstoff | | pro Kapsel |
|---|---|---|
| Hydroxylaminderivat | | 10,0 mg |
| Lactose | | 165,0 mg |
| Maisstärke | | 20,0 mg |
| Talkum | | 5,0 mg |
| | Kapselfüllgewicht | 200,0 mg |

**Patentansprüche**

**1.** Verbindungen der allgemeinen Formel

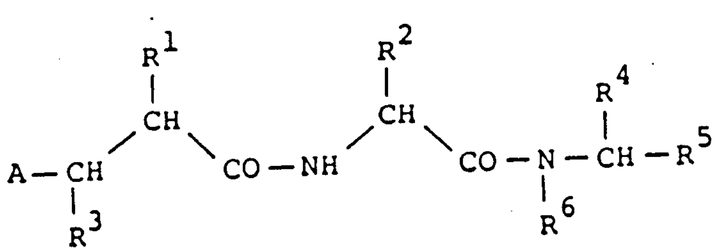

I

worin

A eine Gruppe der Formel HN(OH)-CO- oder HCO-N(OH)-;
$R^1$ eine $C_2$-$C_5$-Alkylgruppe;
$R^2$ die charakterisierende Gruppe einer natürlichen $\alpha$-Aminosäure ist, in der gegebenenfalls vorliegende funktionelle Gruppen geschützt sein können, Aminogruppen acyliert

sein können und Carboxylgruppen amidiert sein können, mit der Bedingung, dass $R^2$ nicht Wasserstoff oder eine Methylgruppe darstellt;

$R^3$ Wasserstoff, eine Amino-, Hydroxy-, Mercapto-, $C_1$-$C_6$-Alkyl-, $C_1$-$C_6$-Alkoxy-, $C_1$-$C_6$-Alkylamino-, $C_1$-$C_6$-Alkylthio- oder Aryl-($C_1$-$C_6$-alkyl)-Gruppe oder Amino-($C_1$-$C_6$-alkyl)-, Hydroxy-($C_1$-$C_6$-alkyl)-, Mercapto-($C_1$-$C_6$-alkyl)- oder Carboxy-($C_1$-$C_6$-alkyl)-Gruppe darstellt, worin die Amino-, Hydroxy-, Mercapto- oder Carboxylgruppe geschützt sein und die Aminogruppe acyliert oder die Carboxylgruppe amidiert sein kann;

$R^4$ Wasserstoff oder eine Methylgruppe;

$R^5$ Wasserstoff oder eine $C_1$-$C_6$-Alkyl, $C_1$-$C_6$-Alkoxy-$C_1$-$C_6$-alkyl, Di($C_1$-$C_6$-alkoxy)-methylen, Carboxy, ($C_1$-$C_6$-Alkyl)carbonyl, ($C_1$-$C_6$-Alkoxy)carbonyl, Arylmethoxycarbonyl, ($C_1$-$C_6$-Alkyl)aminocarbonyl oder Arylaminocarbonylgruppe; und

$R^6$ Wasserstoff oder eine Methylgruppe darstellt; oder

$R^2$ und $R^4$ zusammen eine Gruppe $-(CH_2)_n-$ darstellen, worin n eine Zahl von 4-11 bedeutet; oder

$R^4$ und $R^5$ zusammen eine Trimethylengruppe darstellen;

und pharmazeutisch anwendbare Salze derjenigen Verbindungen, die sauer oder basisch sind.

**2.** Verbindungen gemäss Anspruch 1, worin $R^1$ eine $C_3$- oder $C_4$-Alkylgruppe ist.

**3.** Verbindungen gemäss Anspruch 2, worin $R^1$ n-Propyl, Isobutyl oder sek.Butyl ist.

**4.** Verbindungen gemäss Anspruch 1, 2 oder 3, worin $R^2$ Isopropyl oder Isobutyl, eine geschützte p-Hydroxybenzylgruppe oder eine geschützte oder acylierte 4-Aminobutylgruppe ist.

**5.** Verbindungen gemäss Anspruch 4, worin die geschützte p-Hydroxybenzylgruppe p-Methoxybenzyl ist, die geschützte 4-Aminobutylgruppe 4-tert.Butoxycarbonylamino- butyl und die acylierte 4-Aminobutylgruppe 4-Glycylamino- -butyl oder 4-(4-Carboxy-benzoylamino)butyl ist.

**6.** Verbindungen gemäss Anspruch 1-5, worin $R^3$ Wasserstoff, Methyl oder eine geschützte 4-Aminobutylgruppe ist.

**7.** Verbindungen gemäss Anspruch 6, worin die geschützte 4-Aminobutylgruppe 4-Phthaloylamino-butyl ist.

**8.** Verbindungen gemäss Anspruch 1-7, worin $R^4$ Wasserstoff oder Methyl oder $R^2$ und $R^4$ zusammen eine Gruppe $-(CH_2)_n-$, wobei n eine Zahl von 5-9 ist, darstellt.

**9.** Verbindungen gemäss Anspruch 8, worin $R^4$ Wasserstoff oder Methyl und $R^5$ Wasserstoff oder $C_1$-$C_6$-Alkyl, $C_1$-$C_6$-Alkoxy-$C_1$-$C_6$-alkyl, Di($C_1$-$C_6$)-alkoxy)methylen, Carboxyl oder ($C_1$-$C_6$-Alkoxy)carbonyl ist.

**10.** Verbindungen gemäss Anspruch 9, worin $R^5$ Carboxyl oder ($C_1$-$C_6$-Alkoxy)carbonyl ist.

**11.** Verbindungen gemäss Anspruch 10, worin die ($C_1$-$C_6$-Alkoxy)carbonyl-Gruppe Aethoxycarbonyl ist.

**12.** Verbindungen gemäss Anspruch 8, worin $R^2$ und $R^4$ zusammen eine Gruppe $-(CH_2)_n-$, wobei n eine Zahl von 5 bis 9 und $R^5$ Wasserstoff ist, darstellt.

**13.** Verbindungen gemäss Anspruch 1-12, worin $R^6$ Wasserstoff ist.

**14.** Verbindungen gemäss Anspruch 1-13, worin $R^1$ n-Propyl, Isobutyl oder sek.Butyl; $R^2$ Iso- propyl, Isobutyl, p-Methoxybenzyl, 4-tert.Butoxycarbonylamino-butyl, 4-Glycylaminobutyl oder 4-(4-Carboxy-benzyolamino)-butyl; $R^3$ Wasserstoff oder Methyl oder 4-Phthaloylamino-butyl; $R^4$ Wasserstoff oder Methyl; $R^5$ Carboxyl oder Aethoxycarbonyl und $R^6$ Wasserstoff; oder $R^2$ und $R^4$ zusammen eine Gruppe $-(CH_2)_n-$, wobei n eine Zahl von 5-9, und $R^5$ und $R^6$ Wasserstoff sind, darstellen.

**15.** Verbindungen gemäss Anspruch 1-14, worin das mit $R^1$ verbundene C-Atom (R)-Konfiguration hat; ein die charakterisierende Gruppe einer natürlichen $\alpha$-Aminosäure darstellenden Rest $R^2$ sich von einer L-Aminosäure ableitet; das mit $R^3$ verbundenen C-Atom (S)-Konfiguration hat, wenn $R^3$ $C_1$-$C_6$-Alkyl ist; und das mit $R^4$ verbundene C-Atom (S)-Konfiguration hat, wenn $R^4$ Methyl und $R^5$ Carboxy, ($C_1$-$C_6$-Alkoxy)carbonyl, Arylmethoxycarbonyl, ($C_1$-$C_6$-Alkyl)aminocarbonyl oder Arylcarbonylamino ist.

**16.** [4-(N-Hydroxyamino) 2(R)-isobutylsuccinyl]-L-leucylglycin-äthylester.

**17.** [4-(N-Hydroxyamino) 2(R)-isobutylsuccinyl]-L-leucyl-L-alanin-äthylester.

**18.** [N-Formyl-N-hydroxy 2(RS)-isobutyl-3-aminopropionyl]-L-leucylglycin-äthylester.

**19.** $N^{\alpha}$-[4(N-Hydroxyamino) 2(R)-isobutylsuccinyl]-$N^{\epsilon}$-glycyl-L-lysyl-L-alanin-äthylester.

**20.** [4-(N-Hydroxyamino) 2(R)-isobutylsuccinyl]-3(RS)-aminolaurolactam.

**21.** [4-(N-Hydroxyamino) 3(S)-phthaloylaminobutyl-2(R)-isobutylsuccinyl]-L-leucylglycin-äthylester.

**22.** [4-(N-Hydroxyamino) 2(RS)-isobutylsuccinyl]-L-leucyl-glycin-äthylester;
[4-(N-Hydroxyamino 2(RS)-isobutylsuccinyl]-L-leucyl-glycin-isopentylamid;
[4-(N-hydroxyamino) 2(RS)-isobutylsuccinyl]-L-valylglycin-äthylamid;
[4-(N-Hydroxyamino) 2(RS)-isobutylsuccinyl]-L-leucylglycin-äthylamid;
$N^{\alpha}$-[4-(N-Hydroxyamino) 2(RS)-isobutylsuccinyl]-$N^{\epsilon}$-tert.butoxycarbonyl-L-lysylglycin-äthylamid;
[4-(N-Hydroxyamino) 2(RS)-isobutylsuccinyl]-O-methyl-L-tyrosinylglycin-äthylester;
[4-(N-Hydroxyamino) 2(RS)-isobutylsuccinyl]-O-methyl-L-tyrosinylglycin-äthylamid;
[4-(N-Hydroxyamino) 2(RS)-isobutylsuccinyl]-L-leucyl-L-alanin-äthylester;
[4-(N-Hydroxyamino) 2(RS)-isobutylsuccinyl]-L-leucin-pentylamid;
[4-(N-Hydroxyamino) 2(RS)-isobutylsuccinyl]-L-leucylglycin-isopentylester;
[4-(N-Hydroxyamino) 2(RS)-isobutylsuccinyl]-L-leucin-methylamid;
[4-(N-Hydroxyamino) 2(R)-propylsuccinyl]-L-leucylglycin-ätherester;
[4-(N-Hydroxyamino) 2(RS)-sec.butylsuccinyl]-L-leucylglycin-äthylester;
[4-(N-Hydroxyamino) 2(R)-isobutylsuccinyl]-L-leucyl-L-alanin;
[4-(N-Hydroxyamino) 2(RS)-isobutylsuccinyl]-L-leucylglycin-methylester;
[4-(N-Hydroxyamino) 2(RS)-isobutylsuccinyl]-L-leucylsarcosin-äthylester;
[4-(N-Hydroxyamino) 2(RS)-isobutylsuccinyl]-L-leucyl-L-prolin-äthylester;
[4-(N-Hydroxyamino) 2(RS)-isobutylsuccinyl]-L-leucyl-L-alanin-isopropylester;
[4-(N-Hydroxyamino) 2(RS)-isobutylsuccinyl]-L-leucin-isopropylamid;
[4-(N-Hydroxyamino) 2(RS)-isobutylsuccinyl]-L-leucin-2-oxopropylamid;
[4-(N-Hydroxyamino) 2(RS)-isobutylsuccinyl]-L-leucin-2-methoxyäthylamid;
[4-(N-Hydroxyamino) 2(RS)-isobutylsuccinyl]-L-leucin-2,2-dimethoxyäthylamid;
$N^{\alpha}$-[4-(N-Hydroxyamino) 2(R)-isobutylsuccinyl]-$N^{\epsilon}$-glycyl-L-lysin-methylamid;
$N^{\alpha}$-[4-(N-Hydroxyamino) 2(R)-isobutylsuccinyl]-$N^{\epsilon}$-(4-carboxybenzoyl)-L-lysyl-L-alanin-äthylester;
$N^{\alpha}$-[4-(N-Hydroxyamino) 2(R)-isobutylsuccinyl]-$N^{\epsilon}$-(4-carboxybenzoyl)-L-lysyl-L-alanin;
[4-(N-Hydroxyamino) 2(R)-isobutylsuccinyl]-3(RS)-amino-octahydro-2H-azonin-2-on und
[4-(N-Hydroxyamino) 3(S)-methyl-2(R)-isobutylsuccinyl]-L-leucylglycin-äthylester.

**23.** [N-Formyl-N-hydroxy 2(RS)-isobutyl-3-aminopropionyl]-L-leucyl-L-alanin-äthyl ester und
[N-Formyl-N-hydroxy 2(RS)-isobutyl-3-aminopropionyl]-L-leucin-methylamid.

**24.** Die Verbindungen der Formel I und deren Salze gemäss Definition in Anspruch 1 zur Verwendung als Heilmittel, insbesondere zur Behandlung degenerativer Gelenkerkrankungen.

**25.** Verwendung von Verbindungen der Formel I und deren Salzen gemäss Definition in Anspruch 1 bei der Herstellung von Heilmitteln zur Behandlung von degenerativen Gelenkerkrankungen.

**26.** Pharmazeutische Präparate, enthaltend eine Verbindung der Formel I oder ein Salz davon gemäss Definition in Anspruch 1, und einen pharmazeutischen Trägerstoff.

**27.** Verfahren zur Herstellung von Verbindungen der Formel I und deren Salze gemäss Definition in Anspruch 1, dadurch gekennzeichnet, dass man eine Verbindung der Formel

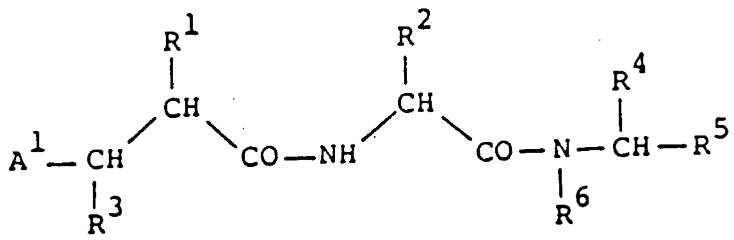

II

worin $A^1$ eine Gruppe der Formel $HN(R^7)$-CO- oder HCO-$N(R^7)$- darstellt, worin $R^7$ eine durch Hydrogenolyse oder Hydrolyse in eine Hydroxygruppe umwandelbare Gruppe ist und $R^1$, $R^2$, $R^3$, $R^4$, $R^5$ und $R^6$ die in Anspruch 1 angegebene Bedeutung haben,
hydrogenolysiert oder hydrolysiert und, gewünschtenfalls, ein saures oder basisches Produkt in ein pharmazeutisch anwendbares Salz überführt.

**28.** Verbindungen der allgemeinen Formel

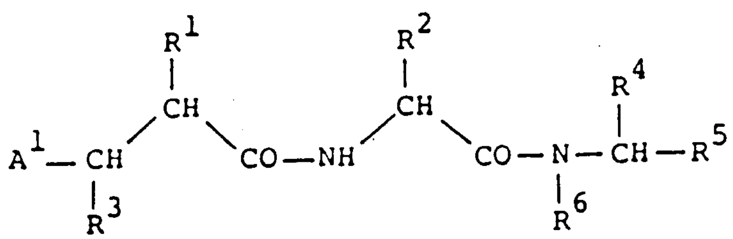

II

worin $A^1$ eine Gruppe der Formel $HN(R^7)$-CO- oder HCO-$N(R^7)$- darstellt, worin $R^7$ eine durch Hydrogenolyse oder Hydrolyse in eine Hydroxygruppe umwandelbare Gruppe ist und $R^1$, $R^2$, $R^3$, $R^4$, $R^5$ und $R^6$ die in Anspruch 1 angegebene Bedeutung haben.

## Claims

**1.** Compounds of the general formula

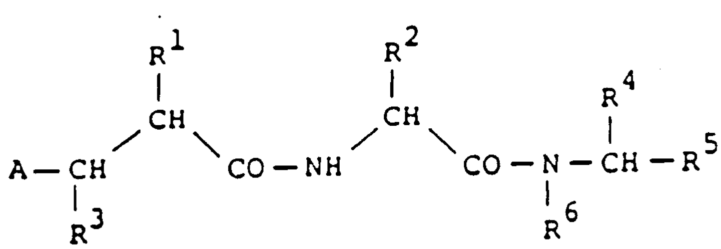

I

wherein

| | |
|---|---|
| A | represents a group of the formula HN(OH)-CO- or HCO-N(OH)-; |
| $R^1$ | represents a $C_2$-$C_5$-alkyl group; |
| $R^2$ | is the characterizing group of a natural α-amino acid in which functional groups optionally present may be protected, amino groups may be acylated and carboxyl groups may be amidated, with the proviso that $R^2$ does not represent hydrogen or an methyl group; $R^3$ represents hydrogen, an amino, hydroxy, mercapto, $C_1$-$C_6$-alkyl, $C_1$-$C_6$-alkoxy, $C_1$-$C_6$-alkylamino, $C_1$-$C_6$-alkylthio or aryl-($C_1$-$C_6$-alkyl) group or an amino-($C_1$-$C_6$-alkyl), hydroxy-($C_1$-$C_6$-alkyl), mercapto-($C_1$-$C_6$-alkyl) or carboxy-($C_1$-$C_6$-alkyl) group in which the amino, hydroxy, mercapto or carboxyl group may be protected and the amino group may be acylated or the carboxyl group may be amidated; |
| $R^4$ | represents hydrogen or a methyl group; |

| | |
|---|---|
| $R^5$ | represents hydrogen or a $C_1$-$C_6$-alkyl, $C_1$-$C_6$-alkoxy-$C_1$-$C_6$-alkyl, di($C_1$-$C_6$-alkoxy)-methylene, carboxy, ($C_1$-$C_6$-alkyl)carbonyl, ($C_1$-$C_6$-alkoxy)carbonyl, arylmethoxycarbonyl, ($C_1$-$C_6$-alkyl)aminocarbonyl or arylaminocarbonyl group; and |
| $R^6$ | represents hydrogen or a methyl group; or |
| $R^2$ and $R^4$ | together represent a group $-(CH_2)_n-$ in which n signifies a number of 4-11; or |
| $R^4$ and $R^6$ | together represent a trimethylene group; |

and pharmaceutically usable salts of those compounds which are acidic or basic.

**2.** Compounds according to claim 1, wherein $R^1$ is a $C_3$- or $C_4$-alkyl group.

**3.** Compounds according to claim 2, wherein $R^1$ is n-propyl, isobutyl or sec.butyl group.

**4.** Compounds according to claim 1, 2 or 3,, wherein $R^2$ is isopropyl or isobutyl, a protected p-hydroxybenzyl group or a protected or acylated 4-aminobutyl group.

**5.** Compounds according to claim 4, wherein the protected p-hydroxybenzyl group is p-methoxybenzyl, the protected 4-aminobutyl group is 4-tert.butoxycarbonylamino-butyl and the acylated 4-aminobutyl group is 4-glycylamino-butyl or 4-(4-carboxy-benzoylamino)-butyl.

**6.** Compounds according to claims 1-5, wherein $R^3$ is hydrogen, methyl group or a protected 4-aminobutyl group.

**7.** Compounds according to claim 6, wherein the protected 4-aminobutyl group is 4-phthaloylamino-butyl.

**8.** Compounds according to claims 1-7, wherein $R^4$ represents hydrogen or methyl or $R^2$ and $R^4$ together represent a group $-(CH_2)_n-$ in which n is a number of 5-9.

**9.** Compounds according to claim 8, wherein $R^4$ is hydrogen or methyl and $R^5$ is hydrogen or $C_1$-$C_6$-alkyl, $C_1$-$C_6$-alkoxy-$C_1$-$C_6$-alkyl, di($C_1$-$C_6$-alkoxy)-methylene, carboxy or ($C_1$-$C_6$-alkoxy)carbonyl.

**10.** Compounds according to claim 9, wherein $R^5$ is carboxy or ($C_1$-$C_6$-alkoxy)carbonyl.

**11.** Compounds according to claim 10, wherein the ($C_1$-$C_6$-alkoxy)carbonyl group is ethoxycarbonyl.

**12.** Compounds according to claim 8, wherein $R^2$ and $R^4$ together represent a group $-(CH_2)_n-$ in which n is a number of 5-9 and $R^5$ is hydrogen.

**13.** Compounds according to any one of claims 1-12, wherein $R^6$ is hydrogen.

**14.** Compounds according to any one of claims 1-13, wherein $R^1$ represents n-propyl, isobutyl or sec.butyl; $R^2$ represents isopropyl, isobutyl, p-methoxybenzyl, 4-tert.butoxycarbonylamino-butyl, 4-glycylaminobutyl or 4-(4-carboxybenzoylamino)-butyl; $R^3$ represents hydrogen or methyl or 4-phthaloylaminobutyl; $R^4$ represents hydrogen or methyl; $R^5$ represents carboxy or ethoxycarbonyl and $R^6$ represents hydrogen; or $R^2$ and $R^4$ together represent a group $-(CH_2)_n-$ in which n represents a number 5-9 and $R^5$ and $R^6$ are hydrogen.

**15.** Compounds according to claims 1-14, wherein the C atom to which $R^1$ is attached has the (R)-configuration; a residue $R^2$ which represents the characterizing group of a natural $\alpha$-amino acid is derived from a L-amino acid; the C atom to which $R^3$ is attached has the (S)-configuration when $R^3$ is $C_1$-$C_6$-alkyl; and the C atom to which $R^4$ is attached has the (S)-configuration when $R^4$ is a methyl group and $R^5$ is carboxy, ($C_1$-$C_6$-alkoxy)carbonyl, arylmethoxycarbonyl, ($C_1$-$C_6$-alkyl)aminocarbonyl or arylcarbonylamino.

**16.** [4-(N-Hydroxyamino) 2(R)-isobutylsuccinyl]-L-leucylglycine ethyl ester.

**17.** [4-(N-Hydroxyamino) 2(R)-isobutylsuccinyl]-L-leucyl-L-alanine ethyl ester.

**18.** [N-Formyl-N-hydroxy 2(RS)-isobutyl-3-aminopropionyl]-L-leucylglycine ethyl ester.

**19.** $N^{\alpha}$-[4-(N-Hydroxyamino) 2(R)-isobutylsuccinyl]-$N^{\epsilon}$-glycyl-L-lysyl-L-alanine ethyl ester.

**20.** [4-(N-Hydroxyamino 2(R)-isobutylsuccinyl]-3(RS)-aminolaurolactam.

**21.** [4-(N-Hydroxyamino) 3(S)-phthaloylaminobutyl-2(R)-isobutylsuccinyl]-L-leucylglycine ethyl ester.

**22.** [4-(N-Hydroxyamino) 2(RS)-isobutylsuccinyl]-L-leucylglycine ethyl ester;
[4-(N-hydroxyamino) 2(RS)-isobutylsuccinyl]-L-leucyl-glycine isopentylamide;
[4-(N-hydroxyamino) 2(RS)-isobutylsuccinyl]-L-valyl-glycine ethylamide;
[4-(N-hydroxyamino) 2(RS)-isobutylsuccinyl]-L-leucyl-glycine ethylamide;
$N^{\alpha}$-[4-(N-hydroxyamino) 2(RS)-isobutylsuccinyl]-$N^{\epsilon}$-tert.butoxycarbonyl-L-lysylglycine ethylamide;
[4-(N-hydroxyamino) 2(RS)-isobutylsuccinyl]-O-methyl-L-tyrosinylglycine ethyl ester;
[4-(N-hydroxyamino) 2(RS)-isobutylsuccinyl]-O-methyl-L-tyrosinylglycine ethylamide;
[4-(N-hydroxyamino) 2(RS)-isobutylsuccinyl]-L-leucyl-L-alanine ethyl ester;
[4-(N-hydroxyamino) 2(RS)-isobutylsuccinyl]-L-leucine pentylamide;
[4-(N-hydroxyamino) 2(RS)-isobutylsuccinyl]-L-leucyl-glycine isopentyl ester;
[4-(N-hydroxyamino) 2(RS)-isobutylsuccinyl]-L-leucine methylamide;
[4-(N-hydroxyamino) 2(R)-propylsuccinyl]-L-leucylglycine ether ester;
[4-(N-hydroxyamino) 2(RS)-sec.butylsuccinyl]-L-leucyl-glycine ethyl ester;
[4-(N-hydroxyamino) 2(R)-isobutylsuccinyl]-L-leucyl-L-alanine;
[4-(N-hydroxyamino) 2(RS)-isobutylsuccinyl]-L-leucyl-glycine methyl ester;
[4-(N-hydroxyamino) 2(RS)-isobutylsuccinyl]-L-leucyl-sarcosine ethyl ester;
[4-(N-hydroxyamino) 2(RS)-isobutylsuccinyl]-L-leucyl-L-proline ethylester;
[4-(N-hydroxyamino) 2(RS)-isobutylsuccinyl]-L-leucyl-L-alanine isopropyl ester;
[4-(N-hydroxyamino) 2(RS)-isobutylsuccinyl]-L-leucine isopropylamide;
[4-(N-hydroxyamino) 2(RS)-isobutylsuccinyl]-L-leucine 2-oxopropylamide;
[4-(N-hydroxyamino) 2(RS)-isobutylsuccinyl]-L-leucine 2-methoxyethylamide;
[4-(N-hydroxyamino) 2(RS)-isobutylsuccinyl]-L-leucine 2,2-dimethoxyethylamide;
$N^{\alpha}$-[4-(N-hydroxyamino) 2(R)-isobutylsuccinyl]-$N^{\epsilon}$-glycyl-L-lysine methylamide;
$N^{\alpha}$-[4-(N-hydroxyamino) 2(R)-isobutylsuccinyl]-$N^{\epsilon}$-(4-carboxybenzoyl)-L-lysyl-L-alanine ethyl ester;
$N^{\alpha}$-[4-(N-hydroxyamino) 2(R)-isobutylsuccinyl]-$N^{\epsilon}$-(4-carboxybenzoyl)-L-lysyl-L-alanine;
[4-(N-hydroxyamino) 2(R)-isobutylsuccinyl]-3(RS)-aminooctahydro-2H-azonin-2-one and
[4-(N-hydroxyamino) 3(S)-methyl-2(R)-isobutylsuccinyl]-L-leucylglycine ethyl ester.

**23.** [N-Formyl-N-hydroxy 2(RS)-isobutyl-3-aminopropionyl]-L-leucyl-L-alanine ethyl ester and
[N-Formyl-N-hydroxy 2(RS)-isobutyl-3-aminopropionyl]-L-leucine methylamide.

**24.** The compounds of formula I and their salts in accordance with the definition in claim 1 for use as medicaments, especially for the treatment of degenerative joint diseases.

**25.** The use of compounds of formula I and their salts in accordance with the definition in claim 1 in the manufacture of medicaments for the treatment of degenerative joint diseases.

**26.** Pharmaceutical preparations containing a compound of formula I or a salt thereof in accordance with the definition in claim 1 on a pharmaceutical carrier material is acidic or basic for used in the control or prevention of degenerative joint diseases.

**27.** A process for the manufacture of the compounds of formula I and their salts in accordance with the definition in claim 1, characterized by hydrogenolyzing or hydrolyzing a compound of the general formula

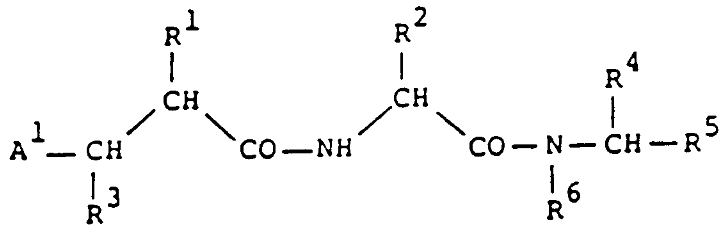

II

wherein $A^1$ represents a group of the formula $HN(R^7)$-CO- or HCO-$N(R^7)$- in which $R^7$ represents a group convertible into a hydroxy group by hydrogenolysis or hydrolysis and $R^1$, $R^2$, $R^3$, $R^4$, $R^5$ and $R^6$ have the significance given in claim 1,
and, if desired, converting an acidic or basic product obtained into a pharmaceutically usable salt.

**28.** Compounds of the general formula

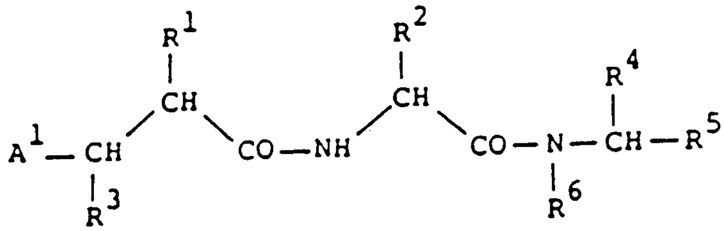

II

wherein $A^1$ represents a group of the formula $HN(R^7)$-CO- or HCO-$N(R^7)$- in which $R^7$ represents a group convertible into a hydroxy group by hydrogenolysis or hydrolysis and $R^1$, $R^2$, $R^3$, $R^4$, $R^5$ and $R^6$ have the significance given in claim 1.

**Revendications**

**1.** Composés de formule générale

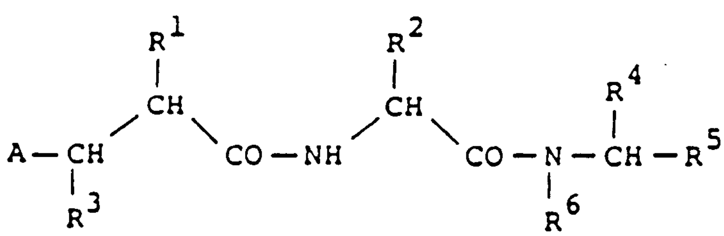

I

où

| | |
|---|---|
| A | est un groupe de formule HN(OH)-CO- ou HCO-N(OH)-; |
| $R^1$ | est un groupe alkyle en $C_2$-$C_5$; |
| $R^2$ | représente le groupe caractérisant un acide aminé en α naturel, dans lequel des groupe fonctionnels éventuellement présents peuvent être protégés, des groupes amino peuvent être acylés et des groupes carboxyle peuvent être amidés, avec pour condition que $R^2$ ne représente pas l'hydrogène ou un groupe méthyle; |
| $R^3$ | représente l'hydrogène, un groupe amino, hydroxy, mercapto, alkyle en $C_1$-$C_6$, alcoxy en $C_1$-$C_6$, alkyl($C_1$-$C_6$)-amino, alkyl($C_1$-$C_6$)-thio ou aryl-alkyle en $C_1$-$C_6$ ou un groupe amino-(alkyle en $C_1$-$C_6$), hydroxy-(alkyle en $C_1$-$C_6$), mercapto-(alkyle en $C_1$ $C_6$) ou carboxy-(alkyle en $C_1$-$C_6$), où le groupe amino, hydroxy, mercapto ou carboxyle peut être protégé ou le groupe amino peut être acylé ou le groupe carboxyle peut être |

amidé;

$R^4$ est l'hydrogène ou un groupe méthyle;

$R^5$ est l'hydrogène ou un groupe alkyle en $C_1$-$C_6$, alcoxy en $C_1$-$C_6$-alkyle en $C_1$-$C_6$, di-(alcoxy en $C_1$-$C_6$)méthylène, carboxy, (alkyl en $C_1$-$C_6$)carbonyle, (alcoxy en $C_1$-$C_6$)-carbonyle, arylméthoxycarbonyle, (alkyl en $C_1$-$C_6$)-aminocarbonyle ou arylaminocarbonyle; et

$R^6$ représente l'hydrogène ou un groupe méthyle; ou

$R^2$ et $R^4$ représentent ensemble un groupe $-(CH_2)_n-$, où n est un nombre de 4 à 11; ou

$R^4$ et $R^5$ représentent ensemble un groupe triméthylène;

et les sels pharmaceutiquement utilisables de ces composés qui sont acides ou basiques.

**2.** Composés selon la revendication 1, où $R^1$ est un groupe alkyle en $C_3$ ou $C_4$.

**3.** Composés selon la revendication 2, où $R^1$ est un groupe n-propyle, isobutyle ou sec-butyle.

**4.** Composés selon l'une quelconque des revendications 1, 2 ou 3, dans lesquels $R^2$ est un groupe isopropyle ou isobutyle, un groupe p-hydroxybenzyle protégé ou un groupe 4-aminobutyle protégé ou acylé.

**5.** Composés selon la revendication 4, dans lesquels le groupe p-hydroxybenzyle protégé est le groupe p-méthoxybenzyle, le groupe 4-aminobutyle protégé est le groupe 4-tert-butoxycarbonylamino-butyle et le groupe 4-aminobutyle acylé est le groupe 4-glycylamino-butyle ou 4-(4-carboxy-benzoylamino)-butyle.

**6.** Composés selon l'une quelconque des revendications 1-5, dans lesquels $R^3$ est l'hydrogène, un groupe méthyle ou un groupe 4-aminobutyle protégé.

**7.** Composés selon la revendication 6, dans lesquels le groupe -aminobutyle protégé est le groupe 4-phtaloylamino-butyle.

**8.** Composés selon l'une quelconque des revendications 1-7, dans lesquels $R^4$ est l'hydrogène ou un groupe méthyle ou $R^2$ et $R^4$ représentent ensemble un groupe $-(CH_2)_n-$, où n est un nombre de 5 à 9.

**9.** Composés selon la revendication 8, dans lesquels $R^4$ est l'hydrogène ou un groupe méthyle et $R^5$ est l'hydrogène ou un groupe alkyle en $C_1$-$C_6$, alcoxy en $C_1$-$C_6$-alkyle en $C_1$-$C_6$, di(alcoxy en $C_1$-$C_6$)-méthylène, carboxyle ou (alcoxy en $C_1$-$C_6$)carbonyle.

**10.** Composés selon la revendication 9, dans lesquels $R^5$ est un groupe carboxyle ou (alcoxy en $C_1$-$C_6$)-carbonyle.

**11.** Composés selon la revendication 10, dans lesquels le groupe (alcoxy en $C_1$-$C_6$)carbonyle est le groupe éthoxycarbonyle.

**12.** Composés selon la revendication 8, dans lesquels $R^2$ et $R^4$ représentent ensemble un groupe $-(CH_2)_n-$ où n est un nombre de 5 à 9 et $R^5$ est l'hydrogène.

**13.** Composés selon l'une quelconque des revendications 1-12, dans lesquels $R^6$ est l'hydrogène.

**14.** Composés selon l'une quelconque des revendications 1-13, dans lesquels $R^1$ est un groupe n-propyle, isobutyle ou sec-butyle; $R^2$ est un groupe isopropyle, isobutyle, p-méthoxybenzyle, 4-tert-butoxycarbonylaminobutyle, 4-glycylaminobutyle ou 4-(4-carboxybenzoylamino)butyle; $R^3$ est l'hydrogène ou un groupe méthyle ou 4-phtaloylamino-butyle; $R^4$ est l'hydrogène ou un groupe méthyle; $R^5$ est un groupe carboxyle ou éthoxycarbonyle et $R^6$ est l'hydrogène; ou $R^2$ et $R^4$ représentent ensemble un groupe $-(CH_2)_n-$, où n représente un nombre de 5 à 9, et $R^5$ et $R^6$ sont l'hydrogène.

**15.** Composés selon l'une quelconque des revendications 1-14, dans lesquels l'atome de carbone lié au groupe $R^1$ a la configuration (R); un reste $R^2$ représentant le groupe caractérisant un acide aminé $\alpha$ naturel dérive d'un acide L-aminé; l'atome de carbone lié à $R^3$ a la configuration (S), lorsque $R^3$ est un

groupe alkyle en $C_1$-$C_6$ ; et l'atome de carbone lié à $R^4$ a la configuration (S), lorsque $R^4$ est un groupe méthyle et $R^5$ un groupe carboxy, (alcoxy en $C_1$-$C_6$)carbonyle, arylméthoxycarbonyle, (alkyl en $C_1$-$C_6$)-carbonyle ou arylcarbonylamino.

**16.** Ester éthylique de [4-(N-hydroxyamino) 2(R)-isobutylsuccinyl]-L-leucylglycine.

**17.** Ester éthylique de [4-(N-hydroxyamino) 2(R)-isobutylsuccinyl]-L-leucyl-L-alanine.

**18.** Ester éthylique de [N-formyl-N-hydroxy 2(RS)-isobutyl-3-aminopropionyl]-L-leucylglycine.

**19.** Ester éthylique de $N^{\alpha}$-[4-(N-hydroxyamino)-2(R)-isobutylsuccinyl]-$N^{\epsilon}$-glycyl-L-lysyl-L-alanine.

**20.** [4-(N-hydroxyamino) 2(R)-isobutylsuccinyl]-3(RS)-aminolaurolactame.

**21.** Ester éthylique de [4-(N-hydroxyamino) 3(S)-phtaloylaminobutyl-2(R)-isobutylsuccinyl]-L-leucylglycine.

**22.** Ester éthylique de [4-(N-hydroxyamino) 2(RS)-isobutylsuccinnyl]-L-leucylglycine;
Isopentylamide de [4-(N-hydroxyamino) 2(RS)-isobutylsuccinyl]-L-leucylglycine;
Ethylamide de [4-(N-hydroxyamino) 2(RS)-isobutylsuccinyl]-L-valylglycine;
Ethylamide de [4-(N-hydroxyamino) 2(RS)-isobutylsuccinyl]-L-leucylglycine;
Ethylamide de $N^{\alpha}$-[4-(N-hydroxyamino) 2(RS)-isobutylsuccinyl]-$N^{\epsilon}$-tert-butoxycarbonyl-L-lysylglycine;
Ester éythylique de [4-(N-hydroxyamino) 2(RS)-isobutylsuccinyl]-O-méthyl-L-tyrosinylglycine;
Ethylamide de [4-(N-hydroxyamino) 2(RS)-isobutylsuccinyl]-O-méthyl-L-tyrosinylglycine;
Ester éthylique de [4-(N-hydroxyamino) 2(RS)-isobutylsuccinyl]-L-leucyl-L-alanine;
Pentylamide de [4-(N-hydroxyamino) 2(RS)-isobutylsuccinyl]-L-leucine;
Ester isopentylique de [4-(N-hydroxyamino) 2(RS)-isobutylsuccinyl]-L-leucylglycine;
Méthylamide de [4-(N-hydroxyamino) 2(RS)-isobutylsuccinyl]-L-leucine;
Ester éthylique de [4-(N-hydroxyamino) 2(RS)-propylsuccinyl]-L-leucylglycine;
Ester éthylique de [4-(N-hydroxyamino) 2(RS)-sec-butylsuccinyl]-L-leucylglycine;
[4-(N-hydroxyamino) 2(R)-isobutylsuccinyl]-L-leucyl-L-alanine;
Ester méthylique de [4-(N-hydroxyamino) 2(RS)-isobutylsuccinyl]-L-leucylglycine;
Ester éthylique de [4-(N-hydroxyamino) 2(RS)-isobutylsuccinyl]-L-leucylsarcosine;
Ester éthylique de [4-(N-hydroxyamino) 2(RS)-isobutylsuccinyl]-L-leucyl-L-proline;
Ester isopropylique de [4-(N-hydroxyamino) 2(RS)-isobutylsuccinyl]-L-leucyl-L-alanine;
Isopropylamide de [4-(N-hydroxyamino) 2(RS)-isobutylsuccinyl]-L-leucine;
2-Oxopropylamide de [4-(N-hydroxyamino) 2(RS)-isobutylsuccinyl]-L-leucine;
2-Méthoxyéthylamide de [4-(N-hydroxyamino) 2(RS)-isobutylsuccinyl]-L-leucine;
2,2-Diméthoxyéthylamide de [4-(N-hydroxyamino) 2(RS)-isobutylsuccinyl]-L-leucine;
Méthylamide de $N^{\alpha}$-[4-(N-hydroxyamino) 2(R)-isobutylsuccinyl]-$N^{\epsilon}$-glycyl-L-lysine;
Ester éthylique de $N^{\alpha}$-[4-(N-hydroxyamino) 2(R)-isobutylsuccinyl]-$N^{\epsilon}$-(4-carboxybenzoyl)-L-lysyl-L-alanine;
$N^{\alpha}$-[4-(N-hydroxyamino) 2(R)-isobutylsuccinyl]-$N^{\epsilon}$ -(4-carboxybenzoyl)-L-lysyl-L-alanine;
[4-(N-hydroxyamino) 2(R)-isobutylsuccinyl]-3-(RS)-amino-octanydro-2H-azonine-2-one et
Ester éthylique de [4-(N-hydroxyamino) 3(S)-méthyl-2(R)-isobutylsuccinyl]-L-leucylglycine.

**23.** Ester éthylique de [N-formyl-N-hydroxy 2(RS)-isobutyl-3-aminopropionyl]-L-leucyl-L-alanine et
Méthylamide de [N-formyl-N-hydroxy 2(RS)-isobutyl-3-aminopropionyl]-L-leucine.

**24.** Composés de formule I et leurs sels selon la définition de la revendication 1 pour une utilisation comme médicament, en particulier pour le traitement des maladies dégénératives des articulations.

**25.** Utilisation des composés de formule I et de leurs sels selon la définition de la revendication 1 dans la préparation de médicaments pour le traitement des maladies dégénératives des articulations.

**26.** Préparation pharmaceutique, contenant un composé de formule I ou un sel de celui-ci selon la définition de la revendication 1, et un support ou vecteur pharmaceutique.

**27.** Procédé pour la préparation de composés de formule I et de leurs sels selon la définition de la revendication 1, caractérisé en ce qu'on hydrogénolyse ou on hydrolyse un composé de formule

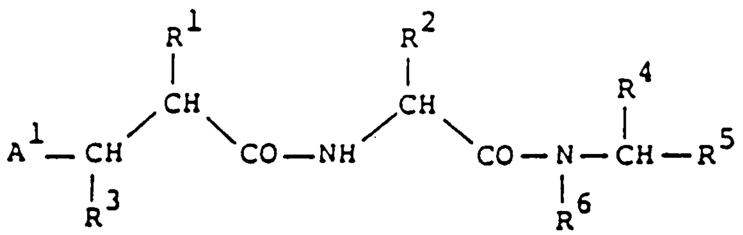

II

où $A^1$ représente un groupe de formule $HN(R^7)$-CO-ou HCO-$N(R^7)$-, dans lequel $R^7$ est un groupe transformable par hydrogénolyse ou hydrolyse en un groupe hydroxy et $R^1$, $R^2$, $R^3$, $R^4$, $R^5$ et $R^6$ ont la signification indiquée dans la revendication 1,
et éventuellement on convertit un produit acide ou basique en un sel pharmaceutiquement utilisable.

**28.** Composés de formule générale

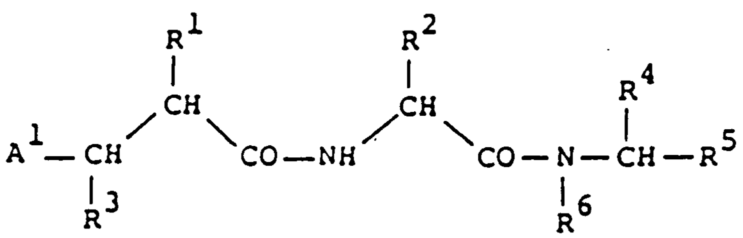

II

où $A^1$ représente un groupe de formule $HN(R^7)$-CO-ou HCO-$N(R^7)$-, dans lequel $R^7$ est un groupe transformable par hydrogénolyse ou hydrolyse en un groupe hydroxy et $R^1$, $R^2$, $R^3$, $R^4$, $R^5$ et $R^6$ ont la signification indiquée dans la revendication 1.